# EUROPÄISCHE PATENTSCHRIFT

(11) **EP 0 757 679 B1**
(45) Veröffentlichungstag und Bekanntmachung des Hinweises auf die Patenterteilung: **21.10.1998**
(21) Anmeldenummer: 95917311.3
(22) Anmeldetag: 12.04.1995
(51) Int. Cl.: C07D 239/42, A01N 47/36, C07D 251/12, C07D 251/16, C07D 251/46, C07D 239/46, C07D 239/52

(54) **ACYLIERTE AMINOPHENYLSULFONYLHARNSTOFFE, VERFAHREN ZU DEREN HERSTELLUNG UND VERWENDUNG ALS HERBIZIDE UND PFLANZENWACHSTUMSREGULATOREN**
ACYLATED AMINOPHENYLSULPHONYLUREAS, PROCESS FOR THEIR PREPARATION AND THEIR USE AS HERBICIDES AND PLANT-GROWTH REGULATORS
AMINOPHENYLSULFONYLUREES ACYLEES, LEUR PROCEDE DE PREPARATION ET LEUR UTILISATION COMME HERBICIDES OU COMME REGULATEURS DE CROISSANCE POUR VEGETAUX

(30) Priorität: 29.04.1994 DE 4415049
(43) Veröffentlichungstag der Anmeldung: 12.02.1997
(73) Patentinhaber: Hoechst Schering AgrEvo GmbH, 13509 Berlin (DE)
(72) Erfinder: SCHNABEL, Gerhard, D-63868 Grosswallstadt (DE); WILLMS, Lothar, D-65719 Hofheim (DE); BAUER, Klaus, D-63456 Hanau (DE); BIERINGER, Hermann, D-65817 Eppstein (DE)
(86) Internationale Anmeldenummer: EP9501344
(87) Internationale Veröffentlichungsnummer: WO9529899

(56) Entgegenhaltungen:
- DE-A- 4 236 902
- US-A- 4 892 946

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß heterocyclisch substituierte Phenylsulfonylharnstoffe, die am Phenylring eine Amino- bzw. eine funktionalisierte Aminogruppe tragen, herbizide und pflanzenwachstumsregulierende Eigenschaften besitzen (EP-A-1515; EP-A-7687 ( = US-A-4,383,113); EP-A-30138 (= US-A-4,394,506); US-A-4,892,946; US-A-4,981,509; EP-A-116518 (= US-A-4,664,695, US-A-4,632,695)).

Außerdem wurden in der deutschen Patentanmeldung P 42 36 902.9 (WO-94/10154) bereits Phenylsulfonylharnstoffe vorgeschlagen, die am Phenylring in 2-Stellung eine Carboxygruppe oder eine von der Carboxygruppe abgeleitete Funktion und in 5-Stellung eine N-Alkyl-N-acyl-aminogruppe aufweisen.

Überraschenderweise wurde nun gefunden, daß bestimmte heterocyclisch substituierte Phenylsulfonylharnstoffe als Herbizide und Pflanzenwachstumsregulatoren besonders gut geeignet sind.

Gegenstand der vorliegenden Erfindung sind Verbindungen der Formel (I) und deren Salze, 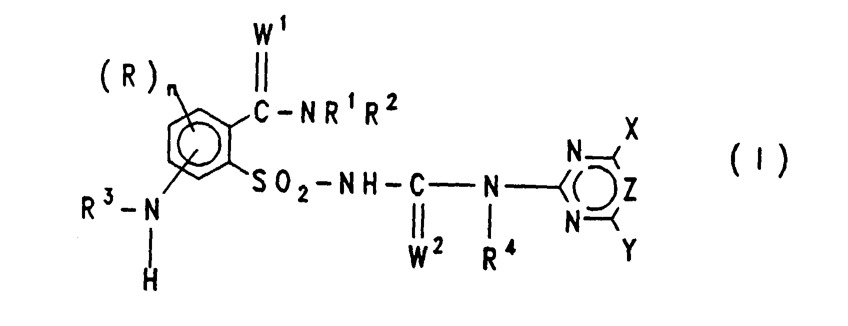 worin
- W¹: ein Sauerstoff- oder Schwefelatom,
- W²: ein Sauerstoff- oder Schwefelatom,
- n: 0, 1, 2 oder 3, vorzugsweise 0 oder 1, insbesondere 0,
- R: Halogen, Alkyl oder Alkoxy, und zwar unabhängig von anderen Substituenten R, wenn n größer 1 ist,
- R¹: Wasserstoff oder einen unsubstituierten oder substituierten Kohlenwasserstoff- oder Kohlenwasserstoffoxyrest,
- R²: Wasserstoff oder einen unsubstituierten oder substituierten Kohlenwasserstoffrest,
oder die Gruppe
- NR¹R²: einen heterocyclischen Ring mit 3 bis 8 Ringatomen, der unsubstituiert oder substituiert ist und das N-Atom der Gruppe NR¹R² als Heteroringatom enthält und noch weitere Heteroringatome enthalten kann,
- R³: einen Acylrest,
- R⁴: Wasserstoff oder einen aliphatischen Kohlenwasserstoffrest,
- X, Y: unabhängig voneinander Halogen, C₁-C₆-Alkyl, C₁-C₆-Alkoxy, C₁-C₆-Alkylthio, wobei jeder der drei letztgenannten Reste unsubstituiert oder durch einen oder mehrere Reste aus der Gruppe Halogen, C₁-C₄-Alkoxy und C₁-C₄-Alkylthio substituiert ist, oder C₃-C₆-Cycloalkyl, C₂-C₆-Alkenyl, C₂-C₆-Alkinyl, C₃-C₆-Alkenyloxy oder C₃-C₈-Alkinyloxy und
- Z: CH oder N bedeuten.

In der Formel (I) und den im folgenden verwendeten Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z. B. Alkyl, Alkenyl, Alkinyl, Cycloalkyl, Cycloalkenyl oder Aryl, vorzugsweise Alkyl, Alkenyl oder Alkinyl mit bis zu 12 C-Atomen oder Cycloalkyl mit 5 oder 6 Ringatomen oder Phenyl; entsprechendes gilt für einen Kohlenwasserstoffoxyrest.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl; Aryloxy bedeutet vorzugsweise ein dem genannten Arylrest entsprechender Oxy-Rest, insbesondere Phenoxy.

Heteroaryl oder ein heteroaromatischer Rest bedeutet ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, aber auch bicyclische oder polycyclische aromatische oder araliphatische Verbindungen, z.B. Chinolinyl, Benzoxazolyl etc. Heteroaryl schließt auch einen heteroaromatischen Ring ein, der vorzugsweise 5- oder 6-gliedrig ist und 1, 2 oder 3 Heteroringatome, insbesondere aus der Gruppe N, O und S enthält. Im substituierten Fall kann der heteroaromatische Ring auch benzokondensiert sein.

Ein heterocyclischer Rest oder Ring kann gesättigt, ungesättigt oder heteroaromatisch sein; er enthält ein oder mehrere Heteroringatome, vorzugsweise aus der Gruppe N, O und S; vorzugsweise ist er 5- oder 6-gliedrig und enthält 1, 2 oder 3 Heteroringatome. Der Rest kann z.B. ein wie oben definierter heteroaromatischer Rest oder Ring sein oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heteroaryl, ein substituierter bicyclischer Rest oder Ring oder ein substituierter bicyclischer Rest, gegebenenfalls mit aromatischen Anteilen, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Mono- und disubstituiertes Amino bedeutet z. B. Alkylamino, Dialkylamino, Acylamino, Arylamino, N-Aryl-N-alkylamino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, z.B. o-, m- und p-Tolyl, Dimethylphenyle, 2-, 3- und 4-Chlorphenyl, 2-, 3- und 4-Trifluor- und-Trichlorphenyl, 2,4-, 3,5-, 2,5- und 2,3-Dichlorphenyl, o-, m- und p-Methoxyphenyl.

Ein Acylrest bedeutet den Rest einer organischen Säure, z. B. den Rest einer Carbonsäure und Reste davon abgeleiteter Säuren wie der Thiocarbonsäure, gegebenenfalls N-substituierter Iminocarbonsäuren, oder der Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie (C₁-C₄-Alkyl)-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der -SO₂-NH-Gruppe oder auch andere acide Wasserstoffatome (z.B. aus COOH u.a.) durch ein für die Landwirtschaft geeignetes Kation ersetzt wird. Diese Salze sind beispielsweise Metallsalze, vorzugsweise Alkali- oder Erdalkalisalze, insbesondere Natrium- und Kaliumsalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Von besonderem Interesse sind erfindungsgemäße Verbindungen der Formel (I) oder deren Salze, worin
- R¹: H oder Alkyl, Alkenyl, Alkinyl, Alkoxy, Alkenoxy, Alkinoxy, Cycloalkyl, Cycloalkenyl oder Phenyl, wobei jeder der neun letztgenannten Reste unsubstituiert oder substituiert ist und insgesamt bis zu 24 C-Atomen, vorzugsweise bis zu insgesamt 12, insbesondere bis zu insgesamt 8 C-Atomen enthält,
- R²: H oder Alkyl, Alkenyl, Alkinyl, wobei jeder der drei letztgenannten Reste unsubstituiert oder substituiert ist und insgesamt bis zu 24 C-Atomen, vorzugsweise bis zu insgesamt 12 C-Atomen, insbesondere bis zu insgesamt 8 C-Atomen enthält,
oder die Gruppe
- NR¹R²: einen unsubstituierten oder substituierten heterocyclischen Ring aus vier bis acht Ringatomen, wobei die Gruppe bis zu insgesamt 18 C-Atomen, vorzugsweise bis zu insgesamt 12 C-Atomen enthält,
- R³: Acyl mit bis zu 24 C-Atomen, vorzugsweise bis zu 12 C-Atomen, insbesondere bis zu 8 C-Atomen,
- R⁴: H oder Alkyl, Alkenyl oder Alkinyl, wobei jeder der 3 letztgenannten Reste bis zu 12 C-Atomen, vorzugsweise bis zu 5 C-Atomen enthält, bedeuten.

Von besonderem Interesse sind erfindungsgemäße Verbindungen der Formel (I) oder deren Salze, worin
- R¹: H, C₁-C₁₂-Alkyl, C₂-C₁₂-Alkenyl, C₂-C₁₂-Alkinyl, C₁-C₁₂-Alkoxy, C₂-C₁₂-Alkenoxy, C₂-C₁₂-Alkinoxy, C₃-C₈-Cycloalkyl oder C₅-C₈-Cycloalkenyl, wobei jeder der acht letztgenannten Reste unsubstituiert oder substituiert ist, beispielsweise durch einen oder mehrere Reste aus der Gruppe Halogen, Cyano, Azido, CO-R⁵, OR⁶, NR⁷R⁸, SR⁹, SO-R¹⁰ und SO₂-R¹¹ substituiert ist, oder unsubstituiertes oder substituiertes Phenyl,
- R²: H, C₁-C₁₂-Alkyl, C₂-C₁₂-Alkenyl oder C₂-C₁₂-Alkinyl, wobei jeder der drei letztgenannten Reste unsubstituiert oder substituiert ist, beispielsweise durch einen oder mehrere Reste aus der Gruppe Halogen, Cyano, Azido, COR¹², OR¹³, NR¹⁴R¹⁵, SR¹⁶, SO-R¹⁷ und SO₂-R¹⁸ substituiert ist,
oder die Gruppe
- NR¹R²: einen heterocyclischen Ring aus vier bis acht Ringatomen, der bis zu zwei weitere Heteroringatome aus der Gruppe N, O und S im Ring enthalten kann und der unsubstituiert oder durch einen oder mehrere Reste aus der Gruppe Halogen, C₁-C₆-Alkyl, NO₂, N₃ und CN substituiert ist,
- R³: CO-R¹⁹, CS-R²⁰, SO₂-R²¹, SO-R²¹ oder C(=NR²¹)-R¹⁹,
- R⁴: H, C₁-C₃-Alkyl, C₂-C₅-Alkenyl oder C₂-C₅-Alkinyl,
- R⁵: H, C₁-C₅-Alkyl, C₂-C₅-Alkenyl, C₂-C₅-Alkinyl, C₁-C₅-Alkoxy, C₂-C₅-Alkenoxy, C₂-C₅-Alkinoxy, NR²²R²³ oder OH,
- R⁶: H, C₁-C₅-Alkyl, C₁-C₅-Haloalkyl, C₂-C₅-Alkenyl, C₂-C₅-Haloalkenyl, C₂-C₅-Alkinyl, C₂-C₅-Haloalkinyl,
- R⁷: H, C₁-C₅-Alkyl, C₂-C₅-Alkenyl, C₂-C₅-Alkinyl, C₁-C₅-Alkoxy, CO-CH₃, CO-H, COOCH₃,
- R⁸: H, C₁-C₅-Alkyl, C₂-C₅-Alkenyl, C₂-C₅-Alkinyl
oder die Gruppe
- NR⁷R⁸: einen heterocyclischen Ring aus vier bis acht Ringatomen, der bis zu zwei weitere Heteroringatome aus der Gruppe N, O und S im Ring enthalten kann und der unsubstituiert oder durch einen oder mehrere Reste aus der Gruppe Halogen, C₁-C₆-Alkyl, NO₂, N₃ und CN substituiert ist,
- R⁹: H, C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl,
- R¹⁰: C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl,
- R¹¹: C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl, C₁-C₅-Alkoxyalkyl, C₃-C₈-Cycloalkyl oder C₅-C₈-Cycloalkenyl,
- R¹²: einen Rest analog R⁵,
- R¹³: einen Rest analog R⁶,
- R¹⁴: einen Rest analog R⁷,
- R¹⁵: einen Rest analog R⁸
oder die Gruppe
- NR¹⁴R¹⁵: eine Gruppe analog NR⁷R⁸,
- R¹⁶: einen Rest analog R⁹,
- R¹⁷: einen Rest analog R¹⁰,
- R¹⁸: einen Rest analog R¹¹,
- R¹⁹: H, C₁-C₁₂-Alkyl, C₂-C₁₂-Alkenyl, C₂-C₁₂-Alkinyl, C₁-C₁₂-Alkoxy, C₁-C₁₂-Alkylthio, C₂-C₁₂-Alkenoxy, C₂-C₁₂-Alkinoxy, C₃-C₈-Cycloalkyl, C₅-C₈-Cycloalkenyl, C₁-C₁₂-Alkylamino, Di-(C₁-C₆-Alkyl)-amino, N-C₁-C₄-Alkoxy-N-C₁-C₄-alkyl-amino, wobei jeder der 13 letztgenannten Reste unsubstituiert oder substituiert ist, beispielsweise durch einen oder mehrere Reste aus der Gruppe Halogen, Cyano, Azido, CO-R²⁴, OR²⁵, NR²⁶R²⁷, SR²⁸, SOR²⁹ und SO₂R³⁰ substituiert ist, oder einen unsubstituierten oder substituierten Phenyl-, Phenoxy- oder Phenylaminorest,
- R²⁰: einen Rest analog R¹⁹,
- R²¹: einen Rest analog R¹¹,
- R²²: H, C₁-C₅-Alkyl, C₂-C₅-Alkenyl, C₂-C₅-Alkinyl, C₁-C₅-Alkoxy, C₂-C₅-Alkenoxy, C₂-C₅-Alkinoxy, wobei jeder der letztgenannten 6 Reste unsubstituiert oder substituiert ist, beispielsweise durch einen oder mehrere Reste aus der Gruppe Halogen, Cyano, Azido, Amino, mono- und disubstituiertes Amino, C₁-C₃-Alkyl, C₁-C₃-Alkoxy, C₁-C₃-Alkylsulfonyl, C₁-C₃-Alkylsulfinyl und C₁-C₃-Alkylthio substituiert ist,
- R²³: H, C₁-C₅-Alkyl, C₂-C₅-Alkenyl, C₂-C₅-Alkinyl, C₁-C₅-Alkoxy, C₂-C₅-Alkenoxy, wobei jeder der 5 letztgenannten Reste unsubstituiert oder substituiert ist, beispielsweise durch einen oder mehrere Reste aus der Gruppe Halogen, Cyano, Azido, Amino, mono- und disubstituiertes Amino, C₁-C₃-Alkoxy, C₁-C₃-Alkylthio, C₁-C₃-Alkylsulfonyl und C₁-C₃-Alkylsulfinyl substituiert ist,
oder die Gruppe
- NR²²R²³: einen heterocyclischen Ring analog NR⁷R⁸, vorzugsweise einen Ring aus 5 oder 6 Ringatomen, der bis zu 2 weitere Heteroringatome aus der Gruppe N, O und S im Ring enthalten kann und der unsubstituiert oder durch einen oder mehrere Reste aus der Gruppe Halogen oder C₁-C₄-Alkyl substituiert ist,
- R²⁴: H, C₁-C₃-Alkyl, C₁-C₃-Alkoxy, NH₂, mono- bzw. disubstituiertes Amino,
- R²⁵: H, C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl,
- R²⁶: H, C₁-C₃-Alkyl, C₁-C₃-Alkoxy, CO-CH₃, CO-H,
- R²⁷: H, C₁-C₃-Alkyl
oder die Gruppe
- NR²⁶R²⁷: eine Gruppe analog NR²²R²³,
- R²⁸: H, C₁-C₃-Alkyl, C₁-C₃-Haloalkyl, C₂-C₅-Alkenyl, C₂-C₅-Haloalkenyl, C₂-C₅-Alkinyl, C₂-C₅-Haloalkinyl,
- R²⁹: C₁-C₅-Alkyl, C₁-C₅-Haloalkyl, C₂-C₅-Alkenyl, C₂-C₅-Haloalkenyl, C₂-C₅-Alkinyl, C₂-C₅-Haloalkinyl,
- R³⁰: analog R²⁹,
- W¹: O, S, vorzugsweise O,
- W²: O, S, vorzugsweise O,
- X, Y: unabhängig voneinander Halogen, C₁-C₄-Alkyl, C₁-C₄-Alkoxy, C₁-C₄-Alkylthio, wobei jeder der drei letztgenannten Reste unsubstituiert oder durch einen oder mehrere Reste aus der Gruppe Halogen, C₁-C₄-Alkoxy und C₁-C₄-Alkylthio substituiert ist, Mono- oder Di(C₁-C₄-alkyl)amino, C₃-C₆-Cycloalkyl, C₃-C₅-Alkenyl oder C₃-C₅-Alkinyloxy und
- Z: CH oder N
bedeuten.

Bevorzugt sind erfindungsgemäße Verbindungen der Formel (I) oder deren Salze, worin
- R¹: H, C₁-C₆-Alkyl, C₂-C₆-Alkenyl, C₂-C₆-Alkinyl, C₁-C₆-Alkoxy, C₂-C₆-Alkenoxy, C₂-C₆-Alkinoxy oder C₅-C₆-Cycloalkyl, wobei jeder der 7 letztgenannten Reste unsubstituiert oder durch einen oder mehrere Reste aus der Gruppe Halogen, Cyano, Azido, CO-R⁵, OR⁶, NR⁷R⁸, SR⁹, SO-R¹⁰ und SO₂-R¹¹ substituiert ist,
oder Phenyl, das unsubstituiert oder durch Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist,
- R²: H, C₁-C₆-Alkyl, C₂-C₆-Alkenyl oder C₂-C₆-Alkinyl, wobei jeder der drei letztgenannten Reste unsubstituiert oder durch einen oder mehrere Reste aus der Gruppe Halogen, Cyano, Azido, COR¹², OR¹³, NR¹⁴R¹⁵, SR¹⁶, SO-R¹⁷ und SO₂-R¹⁸ substituiert ist,
oder die Gruppe
- NR¹R²: einen heterocyclischen Ring aus 5 oder 6 Ringatomen, der bis zu zwei weitere Heteroringatome aus der Gruppe N und O im Ring enthalten kann und der unsubstituiert oder durch einen oder mehrere Reste aus der Gruppe C₁-C₄-Alkyl substituiert ist,
- R³: CO-R¹⁹, CS-R²⁰, SO₂-R²¹ oder C(=NR²¹)-R¹⁹,
- R⁴: H oder CH₃,
- R⁵: H, C₁-C₃-Alkyl, C₁-C₃-Alkoxy, NR²²R²³, OH,
- R⁶: H, C₁-C₄-Alkyl, C₁-C₄-Haloalkyl,
- R⁷: H, C₁-C₃-Alkyl, C₁-C₃-Alkoxy, CO-CH₃, CO-H, COOCH₃,
- R⁸: H, C₁-C₃-Alkyl,
oder die Gruppe
- NR⁷R⁸: einen heterocyclischen Ring aus 5 oder 6 Ringatomen, der bis zu zwei weitere Heteroringatome aus der Gruppe N und O im Ring enthalten kann und der unsubstituiert oder durch einen oder mehrere C₁-C₄-Alkylreste substituiert ist,
- R⁹: H, C₁-C₃-Alkyl, C₁-C₃-Haloalkyl,
- R¹⁰: C₁-C₃-Alkyl, C₁-C₃-Haloalkyl,
- R¹¹: C₁-C₃-Alkyl, C₁-C₃-Haloalkyl, C₁-C₃-Alkoxy-C₁-C₂-alkyl,
- R¹²: einen Rest analog R⁵,
- R¹³: einen Rest analog R⁶,
- R¹⁴: einen Rest analog R⁷,
- R¹⁵: einen Rest analog R⁸
oder die Gruppe
- NR¹⁴R¹⁵: eine Gruppe analog NR⁷R⁸,
- R¹⁶: einen Rest analog R⁹,
- R¹⁷: einen Rest analog R¹⁰,
- R¹⁸: einen Rest analog R¹¹,
- R¹⁹: H, C₁-C₆-Alkyl, C₂-C₆-Alkenyl, C₂-C₆-Alkinyl, C₁-C₆-Alkoxy, C₁-C₆-Alkylthio, C₂-C₆-Alkenoxy, C₂-C₆-Alkinoxy, C₃-C₆-Cycloalkyl, C₁-C₆-Alkylamino, Di-(C₁-C₄-Alkyl)-amino oder N-C₁-C₄-Alkoxy-N-C₁-C₄-alkylamino, wobei jeder der 11 letztgenannten Reste unsubstituiert oder durch einen oder mehrere Reste aus der Gruppe Halogen, Cyano, CO-R²⁴, OR²⁵, NR²⁶R²⁷, SR²⁸, SOR²⁹ und SO₂R³⁰ substituiert ist,
oder einen Phenyl-, Phenoxy- oder Phenylaminorest, der unsubstituiert oder durch Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist,
- R²⁰: einen Rest analog R¹⁹,
- R²¹: einen Rest analog R¹¹,
- R²²: H, C₁-C₄-Alkyl, C₁-C₄-Alkoxy, wobei jeder der 2 letztgenannten Reste unsubstituiert oder durch einen oder mehrere Reste aus der Gruppe Halogen, Cyano, Amino, Mono- und Di-(C₁-C₄-alkyl)-amino, C₁-C₃-Alkoxy, C₁-C₃-Alkylsulfonyl und C₁-C₃-Alkylthio substituiert ist,
- R²³: H, C₁-C₅-Alkyl, C₂-C₅-Alkenyl, C₂-C₅-Alkinyl, C₁-C₅-Alkoxy, C₂-C₅-Alkenoxy, wobei jeder der 5 letztgenannten Reste unsubstituiert oder substituiert ist, beispielsweise durch einen oder mehrere Reste aus der Gruppe Halogen, Cyano, Azido, Amino, mono- und disubstituiertes Amino, C₁-C₃-Alkyl, C₁-C₃-Alkoxy, C₁-C₃-Alkylthio, C₁-C₃-Alkylsulfonyl und C₁-C₃-Alkylsulfinyl substituiert ist,
oder die Gruppe
- NR²²R²³: eine Gruppe analog NR⁷R⁸,
- R²⁴: H, C₁-C₃-Alkyl, C₁-C₃-Alkoxy, NH₂, Mono- und Di-(C₁-C₄-alkyl)-amino,
- R²⁵: H, C₁-C₅-Alkyl, C₁-C₅-Haloalkyl,
- R²⁶: H oder C₁-C₃-Alkyl,
- R²⁷: H oder C₁-C₃-Alkyl
oder die Gruppe
- NR²⁶R²⁷: eine Gruppe analog NR²²R²³,
- R²⁸: H, C₁-C₃-Alkyl,
- R²⁹: C₁-C₅-Alkyl oder C₁-C₅-Haloalkyl,
- R³⁰: analog R²⁹,
- W¹: O, S, vorzugsweise O,
- W²: O, S, vorzugsweise O,

einer der Reste X und Y
   Halogen, C₁-C₂-Alkyl, C₁-C₂-Alkoxy, C₁-C₂-Alkylthio, wobei jeder der drei letztgenannten Reste unsubstituiert oder durch einen oder mehrere Reste aus der Gruppe Halogen, C₁-C₂-Alkoxy und C₁-C₂-Alkylthio substituiert ist, oder Mono- oder Di(C₁-C₂-alkyl)amino,
   vorzugsweise Halogen, Methyl oder Methoxy, und

   der andere der Reste X und Y
   C₁-C₂-Alkyl, C₁-C₂-Haloalkyl, C₁-C₂-Alkoxy, C₁-C₂-Haloalkoxy oder C₁-C₂-Alkylthio,
   vorzugsweise Methyl oder Methoxy,

- Z: CH oder N, vorzugsweise CH,
bedeuten.

Besonders bevorzugt sind erfindungsgemäße Verbindungen der Formel (I) oder deren Salze, worin
- R¹: H, C₁-C₂-Alkyl, C₁-C₂-Alkoxy, Phenyl, das unsubstituiert oder durch einen oder mehrere Reste aus der Gruppe Halogen, C₁-C₂-Alkyl, C₁-C₂-Alkoxy, C₁-C₂-Halogenalkyl und C₁-C₂-Halogenalkoxy substituiert ist,
- R²: H, C₁-C₂-Alkyl oder C₁-C₂-Alkoxy
oder die Gruppe
- NR¹R²: einen heterocyclischen Ring aus 5 oder 6 Ringatomen, der bis zu einem weiteren Heteroringatom aus der Gruppe N und O im Ring enthalten kann und der unsubstituiert oder durch einen oder mehrere C₁-C₂-Alkylreste substituiert ist,
- R³: CO-R¹⁹, CS-R²⁰ oder SO₂-R²¹,
- R⁴: H oder CH₃,
- R¹⁹: H, C₁-C₄-Alkyl, C₂-C₄-Alkenyl, C₂-C₄-Alkinyl, C₁-C₄-Alkoxy, C₁-C₄-Alkylthio, C₂-C₄-Alkenoxy, C₂-C₄-Alkinoxy, C₃-C₆-Cycloalkyl, C₁-C₄-Alkylamino, Di-(C₁-C₄-Alkyl)-amino, N-C₁-C₂-Alkoxy-N-C₁-C₂-alkyl-amino, wobei jeder der 10 letztgenannten Reste unsubstituiert oder durch einen oder mehrere Reste aus der Gruppe Halogen und C₁-C₄-Alkoxy substituiert ist,
oder einen Phenyl- oder Phenoxyrest, der unsubstituiert oder durch Reste aus der Gruppe Halogen, C₁-C₂-Alkyl, C₁-C₂-Alkoxy, C₁-C₂-Halogenalkyl und C₁-C₂-Halogenalkoxy substituiert ist,
- R²⁰: einen Rest analog R¹⁹ und
- R²¹: C₁-C₃-Alkyl, C₁-C₃-Haloalkyl, C₁-C₃-Alkoxy-C₁-C₂-alkyl bedeuten.

Bei den erfindungsgemäßen Verbindungen der Formel (I) sind solche bevorzugt, in denen die Gruppe der Formel NHR³ am Phenylrest in para-Stellung zur Gruppe CW¹-NR¹R² und in meta-Stellung zur SO₂-Gruppe steht.

Bevorzugt sind auch erfindungsgemäße Verbindungen der Formel (I), die durch eine Kombination von obengenannten jeweils bevorzugt Resten gekennzeichnet sind.

Weiterer Gegenstand der vorliegenden Erfindung sind Verfahren zur Herstellung der erfindungsgemäßen Verbindungen der Formel (I) oder deren Salze, dadurch gekennzeichnet, daß man
a) eine Verbindung der Formel (II) 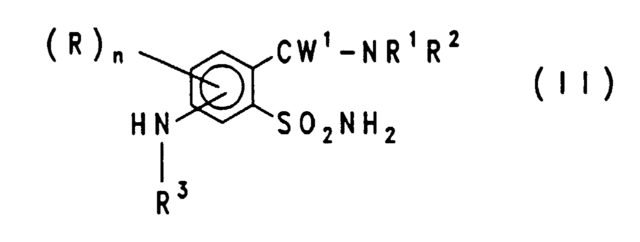 mit einem heterocyclischen Carbamat der Formel (III), 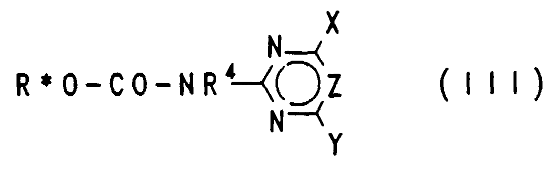 worin R* unsubstituiertes oder substituiertes Phenyl oder C₁-C₄-Alkyl bedeutet, umsetzt oder
b) ein Sulfonylisocyanat der Formel (IV) 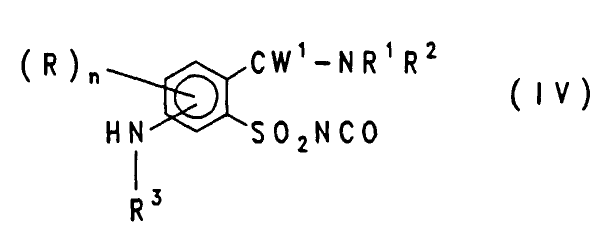 mit einem heterocyclischen Amin der Formel (V) 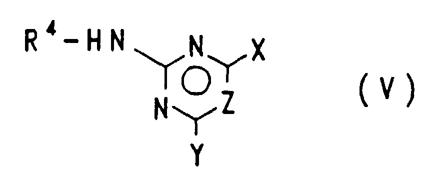 umsetzt oder
c) ein Sulfochlorid der Formel (Vl) 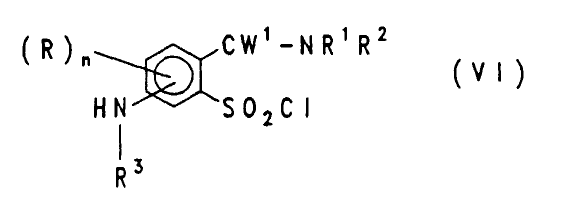 mit einem heterocyclischen Amin der genannten Formel (V) in Gegenwart eines Cyanats, z.B. eines Alkalimetallcyanats wie Natrium- oder Kaliumcyanat, umsetzt oder
d) ein Sulfonamid der genannten Formel (II) mit einem (Thio-)lsocyanat der Formel (VII) 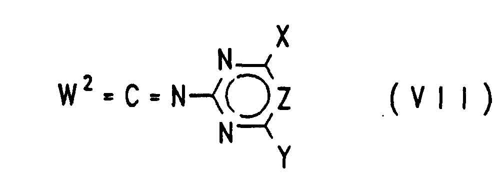 in Gegenwart einer geeigneten Base, wie z.B. Kaliumcarbonat oder Triethylamin, umsetzt,
wobei in den obigen Formeln (II) bis (VII) die Reste R, R¹, R², R³, R⁴, W¹, W², X, Y und Z sowie der Index n wie in Formel (I) definiert sind und wobei in den Varianten a) - c) zunächst Verbindungen der Formel (I) erhalten werden, in denen W² ein Sauerstoffatom bedeutet.

Die Sulfonamide (II), die Sulfonylisocyanate (IV) und die Sulfonylchloride (VI) sind neue Verbindungen. Sie und ihre Herstellung sind ebenfalls Gegenstand der Erfindung.

Die Umsetzung der Verbindungen der Formel (II) und (III) erfolgt vorzugsweise basenkatalysiert in inerten Solventien, wie z. B. Dichlormethan, Acetonitril, Dioxan oder THF, bei Temperaturen von -10° C bis zum Siedepunkt des jeweiligen Lösungsmittels. Als Basen werden dabei beispielsweise organische Aminbasen wie 1,8-Diazabicyclo[5.4.0]undec-7-en (DBU), insbesondere im Falle R* = (subst.) Phenyl (vgl. EP-A-44807), oder Trimethyl- oder Triethylaluminium, letztere insbesondere im Fall R* = Alkyl (vgl. EP-A-166 516), verwendet.

Man erhält die Verbindungen der Formel (II) z.B. aus N-(t-Butyl)-sulfonamiden der Formeln (VIII), (IX) und (X) (siehe Formel VIII*, Z* = R³NH-)

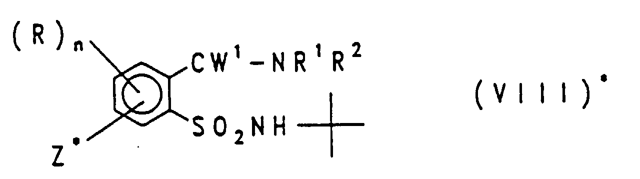

Z* = NHR³ (VIII)

Z* = NH₂ (IX)

Z* = NO₂ (X)

Ausgehend von Verbindungen der Formel (VIII), worin R, n, R¹, R², R³ und W¹ wie in Formel (I) definiert sind, erhält man Verbindungen der Formel (II) durch Umsetzung mit einer starken Säure. Als starke Säure kommen z.B. Mineralsäuren, wie H₂SO₄ oder HCI, oder starke organische Säuren, wie Trifluoressigsäure, in Frage. Die Abspaltung der t-Butylgruppe erfolgt bei Temperaturen zwischen -20°C und der jeweiligen Rückflußtemperatur des Reaktionsgemisches, vorzugsweise bei 0°C bis 40°C. Die Umsetzung kann in Substanz oder auch in einem Solvens, wie z.B. Dichlormethan oder Trichlormethan, durchgeführt werden.

Die Verbindungen der Formel (VIII) werden z.B. aus den Anilin-Derivaten der Formel (IX) (siehe Formel (VIII), Z* = NH₂) durch Umsetzung mit geeigneten Elektrophilen wie z.B. Säurechloriden, Säureanhydriden, Isocyanaten, Thioisocyanaten, Sulfochloriden oder Amidosulfochloriden erhalten (vgl. hierzu: A.L.J. Beckniter in J. Zabicky, "The Chemistry of Amides", S. 73-185, Interscience, New York, 1970; E.J. Corey et al., Tetrahedron.Lett. 1978, 1051; H.J. Saunders, R.J. Slocombe, Chem. Rev. 43, 203 (1948); S. Ozaki, Chem. Rev. 72, 457, 469 (1972); G. Zölß, Arzneim.-Forsch. 33, 2 (1983); Houben-Weyl-Hagemann, "Methoden der organischen Chemie", 4. Aufl. Bd. E4, S. 485 ff., Thieme Verlag Stuttgart, 1983; J. Golinsky, M. Mohasza, Synthesis 1978, 823; Houben-Weyl-Müller, "Methoden der organischen Chemie", 4. Aufl. Bd. IX, S. 338-400 und 605-622, Thieme Verlag Stuttgart, 1955; Houben-Weyl-Klarmann, "Methoden der organischen Chemie", 4. Aufl. Bd. E 11/2, S. 1020-22, Thieme Verlag Stuttgart, 1985; S. Krishnamurthey, Tetrahedron Lett. 23, 3315 (1982).).

Die genannten Aniline (IX) werden nach literaturbekannten Verfahren z.B. durch Reduktion der Nitrogruppen aus den Verbindungen (X) (siehe Formel (VIII), Z* = NO₂), z.B. durch katalytische Hydrierung oder durch Reduktion mit Eisen in essigsaurem Medium, erhalten (vgl. hierzu: H. Berrie, G.T. Neuhold, F.S. Spring, J. Chem. Soc. 1952, 2042; M. Freifelder, "Catalytic Hydrogenation in Organic Synthesis: Procedures and Commentary", J. Wiley and Sons, New York (1978), Kap. 5).

Die Verbindungen der Formel (X) lassen sich ausgehend von den Benzoaten der Formel (Xl) durch Amidierung gewinnen: 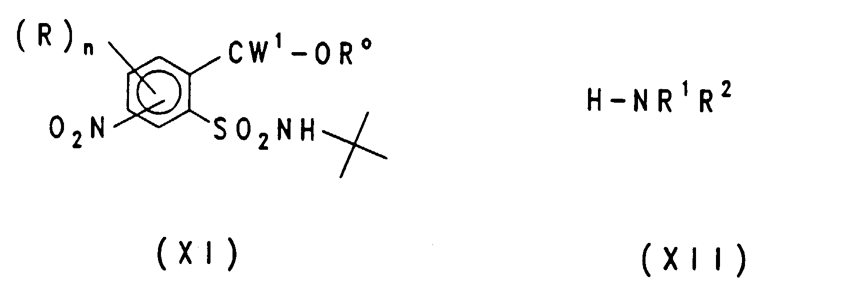 Die Amidbildung erfolgt durch Reaktion der Benzoate (XI) (R^{o} = Alkyl) mit den Aminen der Formel (XII).Bei den Verbindungen der Formel (Xl) und (XII) handelt es sich um literaturbekannte bzw. käufliche Edukte (vgl. z.B. deutsche Patentanmeldung P 42 36 902.9), oder die Verbindungen können analog allgemein bekannten Verfahren hergestellt werden.

Die Amide der Formeln (VIII), (IX) und (X) sind neue Verbindungen. Sie und ihre Herstellung sind ebenfalls Gegenstand der Erfindung.

Einen alternativen Zugang zu Verbindungen (Xl) (W¹ =O) bietet die folgende Synthesesequenz, die im folgenden am Beispiel der Verbindungen mit n = 0 erläutert ist.
Ausgehend von 2-Amino-nitrobenzoesäuren (XIII), beispielsweise 2-Amino-4-nitrobenzoesäure (Xllla), erhält man die entsprechenden Benzoesäureester (XIV) (R^{o} = Alkyl) durch klassische Veresterung mit den entsprechenden Alkoholen (R°OH) und einer geeigneten Säure, wie z.B. H₂SO₄.

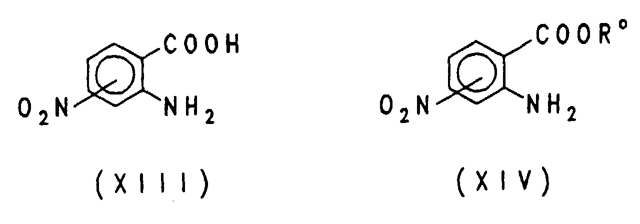

Durch Diazotierung der Aminogruppe und nachfolgender Umsetzung mit SO₂/CuCl₂ erhält man das Sulfochlorid (XV) (Analog Meerwein, Chem. Ber. 90 (1957) 841-852).

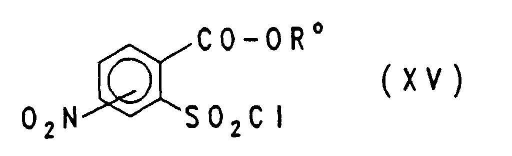

Die Ammonolyse von Verbindungen der Formel (XV) mit t-Butylamin führt zu den Sulfonamiden der Formel (Xl).

Die für die Umsetzung der Verbindungen (II) nach Variante a) benötigten Carbamate der Formel (III) sind literaturbekannt oder lassen sich analog bekannten Verfahren herstellen (vgl. EP-A-70 804 oder US-A-4,480,101).

Die Phenylsulfonylisocyanate der Formel (IV) lassen sich z.B. analog den Verfahren aus EP-A-184 385 aus Verbindungen der Formel (II), z.B. mit Phosgen, herstellen.

Die Umsetzung der Verbindungen (IV) mit den Aminoheterocyclen der Formel (V) führt man vorzugsweise in inerten, aprotischen Lösungsmitteln, wie z.B. Dioxan, Acetonitril oder Tetrahydrofuran bei Temperaturen zwischen 0°C und der Siedetemperatur des Lösungsmittels durch.

Die Umsetzung der Sulfochloride (Vl) mit den Aminoheterocyclen der Formel (V) und Cyanaten wie Natriumcyanat und Kaliumcyanat erfolgt z. B. in aprotischen Solventien, wie z.B. Acetonitril, gegebenenfalls in Gegenwart von Basen, z. B. 0,5 bis 2 Äquivalenten Base, oder in basischen aprotischen Solventien bei Temperaturen zwischen -10°C und 100°C, vorzugsweise -10°C und 60°C, insbesondere bei 15°C bis 40°C. Als Base oder basische aprotische Solventien kommen z.B. Pyridin, Picolin oder Lutidin oder eine Mischung aus diesen in Betracht (vgl. US-A-5,157,119).

Die (Thio-)lsocyanate der Formel (VII) sind nach literaturbekannten Verfahren erhältlich (EP-A-232067, EP-A-166516). Die Umsetzung der (Thio-)lsocyanate (VII) mit Verbindungen (II) erfolgt bei -10 °C bis 100 °C, vorzugsweise 20 bis 100 °C, in einem inerten aprotischen Lösungsmittel, wie z. B. Aceton oder Acetonitril, in Gegenwart einer geeigneten Base, z. B. N(C₂H₅)₃ oder K₂CO₃.

Die Salze der Verbindungen der Formel (I) werden vorzugsweise in inerten Lösungsmitteln wie z. B. Wasser, Methanol, Aceton, Dichlormethan, Tetrahydrofuran, Toluol oder Heptan, bei Temperaturen von 0 bis 100° C hergestellt. Geeignete Basen zur Herstellung der erfindungsgemäßen Salze sind beispielsweise Alkalicarbonate, wie Kaliumcarbonat, Alkali- und Erdalkalihydroxide, wie NaOH, KOH und Ca(OH)₂, Ammoniak oder eine geeignete Aminbase, wie Triethylamin oder Ethanolamin. Als Säuren zur Salzbildung eignen sich z.B. HCI, HBr, H₂SO₄ oder HNO₃.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) oder deren Salze weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchstauchung, eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, anderen Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I) oder deren Salze.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff, versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d. h. 2-Chlor-N,N-di-2-propenylacetamid; CDEC, d. h. Diethyldithiocarbaminsäure-2-chlorallylester; CGA 184927, d. h. 2-[4-[(5-Chlor-3-fluor-2-pyridinyl)-oxy]-phenoxy]-propansäure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmedipham; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; F6285, d. h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosaten; haloxyfop und dessen Esterderivate; hexazinone; Hw 52, d. h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyfluorfen; paraquat; pebulate; pendimethalin; perfluidone; phenmedipham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d. h. 2-[4-Chlor-2-fluor-5-(2-propynyloxy)-phenyl]-4,5,6,7-tetrahydro-2H-indazol; S 482, d. h. 2-[7-Fluor-3,4-dihydro-3-oxo-4-(2-propynyl)-2H-1,4-benzoxazin-6-yl]-4, 5,6,7-tetrahydro-1H-isoindol-1,3(2H)-dion; secbumeton; sethoxydim; siduron; simazine; simetryn; SN 106279, d. h. 2-[[7-[2-Chlor-4-(trifluor-methyl)-phenoxy]-2-naphthalenyl]-oxy]-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d. h. 5-Phenoxy-1-[3-(trifluormethyl)-phenyl]-1H-tetrazol.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser, und anschließend auf die Pflanzen, Pflanzenteile oder den landwirtschaftlich oder industriell genützten Boden, auf dem die Pflanzen stehen oder in dem sie heranwachsen oder als Saat vorliegen, appliziert. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### a) 2-Amino-4-nitro-benzoesäuremethylester

Ein Gemisch aus 118,8 g 2-Amino-4-nitro-benzoesäure, 1000 ml Methanol und 120 ml konz. Schwefelsäure wird 16 h zum Sieden erhitzt. Unter reduziertem Druck wird das Gemisch eingeengt, in Essigsäureethylester aufgenommen und mit gesättigter NaHCO₃-Lösung gewaschen. Nach dem Trocknen über Na₂SO₄ wird die organische Phase eingeengt. Man erhält so 114,2 g (89 %) 2-Amino-4-nitro-benzoesäuremethylester mit einem Schmelzpunkt (Fp.) von 156-158°C.

### b) 2-Chlorsulfonyl-4-nitro-benzoesäuremethylester

Zu einer Suspension aus 1300 ml Eisessig, 516 ml konzentrierter Salzsäure und 441,7 g (1,689 Mol) 2-Amino-4-nitrobenzoesäuremethylester (Bsp. a) wird bei einer Temperatur zwischen 13 und 15°C eine Lösung aus 122,3 g Natriumnitrit in 180 ml Wasser zugetropft. Nach 30 min. Rühren tropft man dieses Reaktionsgemisch zu einer mit SO₂ gesättigten Lösung aus 17 g Kupfer-II-chlorid und 1300 ml Eisessig bei ca. 26°C zu. Nach beendeter Gasentwicklung gießt man auf Eiswasser, saugt das ausgefallene Sulfochlorid ab, wäscht mit Wasser und erhält nach dem Trocknen 354,3 g 2-Chlorsulfonyl-4-nitrobenzosäuremethylester;
Fp.: 88-90°C.

### c) N-tert.-Butyl-2-methoxycarbonyl-5-nitro-benzolsulfonamid

Zu einer Lösung aus 384 g 2-Chlorsulfonyl-4-nitrobenzoesäuremethylester (Bsp. b) in 1500 ml Ethylacetat werden bei 0°C 206 g tert.-Butylamin zugetropft. Anschließend läßt man das Reaktionsgemisch auf Raumtemperatur aufwärmen und rührt 1 h bei dieser Temperatur. Nach dem Waschen mit verdünnter Salzsäure und Wasser wird die organische Phase über MgSO₄ getrocknet und eingeengt. Der Rückstand wird mit Diisopropylether ausgerührt;
Ausbeute an Sulfonamid: 377,6 g; Fp.: 122-124°C.

### d) N-tert.-Butyl-2-dimethylaminocarbonyl-5-nitro-benzolsulfonamid

Zu 115 g N-tert.-Butyl-2-methoxycarbonyl-5-nitro-benzolsulfonamid (Bsp. c) in 1500 ml Methanol werden ca. 200 g Dimethylamin eingegast. Das Gemisch wird eine Woche bei ca. 35°C gerührt. Man engt die Lösung unter reduziertem Druck ein, nimmt den Rückstand in Ethylacetat auf und wäscht nacheinander mit verdünnter Salzsäure, gesättigter NaHCO₃-Lösung und gesättigter NaCl-Lösung. Nach dem Trocknen über MgSO₄ wird die org. Phase eingeengt. Man erhält 92 g an N-tert.-Butyl-2-dimethylaminocarbonyl-5-nitro-benzolsulfonamid;
Fp.: 138-141°C.

### e) 5-Amino-N-tert.-butyl-2-dimethylaminocarbonyl-benzolsulfonamid

Zu einer Suspension aus 22,0 g N-tert.-Butyl-2-dimethylaminocarbonyl-5-nitro-benzolsulfonamid (Bsp. d), 18,2 g Ammoniumchlorid, 70 ml Wasser und 150 ml Methanol werden 23,6 g Zink-Pulver zugesetzt. Anschließend wird das Gemisch bei 50°C gerührt. Nach beendeter Reaktion wird vom Feststoff abfiltriert. Nach dem Waschen des Feststoffes mit Ethylacetat wird das Filtrat eingeengt, der Rückstand in Ethylacetat aufgenommen und mit Wasser gewaschen. Nach dem Trocknen über MgSO₄ wird die organische Phase eingeengt und der Rückstand wird mit Diisopropylether gewaschen; Ausbeute: 17,5 g; Fp.: 205-208°C.

### f) N-tert.-Butyl-2-dimethylaminocarbonyl-5-methoxycarbonylaminobenzolsulfonamid

Zu einer Suspension aus 1,50 g 5-Amino-N-tert.-butyl-2-dimethylaminocarbonyl-benzolsulfonamid (Bsp. e) und 1,46 g NaHCO₃ in 50 ml CH₃CN werden bei 0°C 0,47 g Chlorameisensäuremethylester zugetropft. Nach beendeter Reaktion wird das Reaktionsgemisch in Ethylacetat aufgenommen, mit 1N Salzsäure gewaschen, über MgSO₄ getrocknet und eingeengt. Man erhält so 1,48 g an N-tert.-Butyl-2-dimethylaminocarbonyl-5-methoxycarbonylamino-benzolsulfonamid;
Fp.: 184-188°C.

### g) N-tert.-Butyl-2-dimethylaminocarbonyl-5-formylamino-benzolsulfonamid

Ein Gemisch aus 0,34 ml Ameisensäure und 0,70 ml Essigsäureanhydrid wird 2 h auf 50°C erwärmt und mit einer Lösung aus 0,85 g 5-Amino-N-tert.-butyl-2-dimethylaminocarbonyl-benzolsulfonamid (Bsp. e) und 3,5 ml Dimethylformamid (DMF) versetzt. Nach 4 h wird das Gemisch in Ethylacetat aufgenommen und nacheinander mit verdünnter Salzsäure und gesättigter NaHCO₃-Lösung gewaschen. Nach dem Trocknen über MgSO₄ und dem Einengen der organischen Phase erhält man 0,88 g einer hochviskosen Substanz, die ohne weitere Reinigung in Folgereaktionen eingesetzt wird (Bsp. j).

### h) N-tert.-Butyl-2-dimethylaminocarbonyl-5-propionylamino-benzolsulfonamid

0,85 g 5-Amino-N-tert.-butyl-2-dimethylaminocarbonyl-benzolsulfonarnid (Bsp. e) wird bei 0°C in 3,5 ml DMF gelöst und mit 0,28 g Propionsäurechlorid und 0,50 ml Triethylamin versetzt. Nach 1 h Rühren bei 5°C wird das Gemisch in Ethylacetat aufgenommen und nacheinander mit verdünnter Salzsäure und Wasser gewaschen. Nach dem Trocknen über MgSO₄ und dem Einengen der organischen Phase werden 0,60 g einer hochviskosen Substanz erhalten, die ohne weitere Reinigung in Folgereaktionen eingesetzt wird (Bsp. k).

### i) 2-Dimethylaminocarbonyl-5-methoxycarbonylamino-benzolsulfonamid

1,48 g N-tert.-Butyl-2-dimethylaminocarbonyl-5-methoxycarbonylaminobenzolsulfonamid (Bsp. f) werden 18 h in 25 ml Trifluoressigsäure gerührt. Nach dem Abdestillieren der Säure wird der Rückstand in Toluol suspendiert. Nach dem erneuten Einengen erhält man 1,40 g des Sulfonamids;
Fp.: 75-77°C.

### j) 2-Dimethylaminocarbonyl-5-formylamino-benzolsulfonamid

Analog Bsp. i werden 0,85 g N-tert.-Butyl-2-dimethylamino-carbonyl-5-formylamino-benzolsulfonamid (Bsp. g) mit 10 ml Trifluoressigsäure umgesetzt. Man erhält so 0,88 g einer hochviskosen Masse, die ohne weitere Reinigung in die unter Bsp. m beschriebene Umsetzung eingesetzt wird.

### k) 2-Dimethylaminocarbonyl-5-propionylamino-benzolsulfonamid

Analog Bsp. i werden 0,80 g N-tert.-Butyl-2-dimethylarnino-carbonyl-5-propionylamino-benzolsulfonamid mit 10 ml Trifluoressigsäure umgesetzt. Man erhält so 0,80 g einer hochviskosen Masse, die ohne weitere Reinigung in die unter Bsp. n) beschriebene Reaktion eingesetzt wird.

### l) N-[(4,6-Dimethoxypyrimidin-2-yl]-aminocarbonyl]-2-dimethylaminocarbonyl-5-methoxycarbonylamino-benzolsulfonamid (vgl. Bsp. 53 aus Tabelle 1)

Eine Suspension aus 1,40 g 2-Dimethylaminocarbonyl-5-methoxycarbonylamino-benzolsulfonamid (Bsp. i) und 1,28 g 4,6-Dimethoxy-2-phenoxycarbonylamino-pyrimidin in 30 ml CH₃CN wird mit DBU bei 0°C versetzt. Anschließend läßt man die Reaktionstemperatur langsam auf Raumtemperatur steigen. Nach dem Abdestillieren des Solvens wird der Rückstand in Wasser aufgenommen und mit Diethylether gewaschen. Nach dem Ansäuern der wäßrigen Phase mit konz. Salzsäure wird der abgeschiedene Sulfonylharnstoff mit Methanol und Diisopropylether gewaschen und dann getrocknet. Ausbeute: 1,45 g als farblose Feststoff mit Fp. 181-182°C (Zers.).

### m) N-[(4,6-Dimethoxypyrimidin-2-yl)-aminocarbonyl]-2-dimethylaminocarbonyl-5-formylamino-benzolsulfonamid (vgl. Bsp. 8 aus Tabelle 1)

Analog Bsp. I) werden 0,88 g 2-Dimethylaminocarbonyl-5-formyl-aminobenzolsulfonamid (Bsp. j) mit 0,89 g 4,6-Dimethoxy-2-phenoxycarbonylamino-pyrimidin und 0,98 g DBU in 10 ml CH₃CN umgesetzt. Man erhält 0,69 g des kristallinen Sulfonylharnstoffes mit
Fp.: 126-127°C (Zers.).

### n) N-[(4,6-Dimethoxypyrimidin-2-yl)-aminocarbonyl]-2-dimethylaminocarbonyl-5-propionylamino-benzolsulfonamid (vgl. Bsp. 22 aus Tabelle 1)

Analog Bsp. I) werden 0,80 g 2-Dimethylaminocarbonyl-5-propionylaminobenzolsulfonamid mit 0,74 g 4,6-Dimethoxy-2-phenoxycarbonylaminopyrimidin und 0,82 g DBU in 10 ml CH₃CN umgesetzt. Man erhält 0,68 g des kristallinen Sulfonylharnstoffes mit Fp. 135-140°C (Zers.).

### o) N-[(4,6-Dimethoxypyrimidin-2-yl)-aminocarbonyl]-2-dimethylaminocarbonyl-5-methoxycarbonylamino-benzolsulfonamid-Natriumsalz (vgl. Bsp. 21 aus Tabelle 2)

Zu einem Gemisch aus 0,93 g N-[(4,6-Dimethoxypyrimidin-2-yl)aminocarbonyl]-2-dimethylaminocarbonyl-5-methoxycarbonyl-aminobenzolsulfonamid in 20 ml CH₃CN setzt man 1,85 ml 1N Natronlauge zu. Nach dem sich eine klare Lösung gebildet hat, wird das Gemisch unter reduziertem Druck eingeengt. Der Rückstand wird mit wenig Diisopropylether verrührt. Man erhält so 0,82 g des Salzes mit
Fp.: 187-191°C (Zers.)

### p) N-[(4,6-Dimethoxypyrimidin-2-yl)-aminocarbonyl]-2-dimethylaminocarbonyl-5-formylamino-benzolsulfonamid-Natriumsalz (vgl. Bsp. 1 aus Tabelle 2)

Analog Bsp. o) werden 0,30 g N-[(4,6-Dimethoxypyrimidin-2-yl)aminocarbonyl]-2-dimethylaminocarbonyl-5-formylamino-benzolsulfonamid (Bsp. m) und 0,65 ml 1N Natronlauge in 4 ml Methanol und 4 ml CH₂Cl₂ umgesetzt. Man erhält 0,32 g des Salzes;
Fp.: 205°C (Zers.)

### q) N-[(4,6-Dimethoxypyrimidin-2-yl)-aminocarbonyl]-2-dimethylaminocarbonyl-5-propionylamino-benzolsulfonamid-Natriumsalz (vgl. Bsp. 12 aus Tabelle 2)

Analog Bsp. o) werden 0,30 g N-[(4,6-Dimethoxypyrimidin-2-yl)aminocarbonyl]-2-dimethylamino-carbonyl-5-propionylaminobenzolsulfonamid (Bsp. n) und 0,60 ml 1N Natronlauge in 4 ml Methanol und 4 ml CH₂Cl₂ umgesetzt. Man erhält so 0,31 g des Salzes;
Fp.: 212°C (Zers.)

Die in der nachfolgenden Tabelle 1 beschriebenen Verbindungen werden nach bzw. analog den obigen Beispielen I) bis n) erhalten; die in der nachfolgenden Tabelle 2 beschriebenen Verbindungen nach bzw. analog den obigen Beispielen o) bis q).

### Abkürzungen:

Fp. = Schmelzpunkt
Et = Ethyl
Me = Methyl
Pr = ⁿPr = n-Propyl
ⁱPr = Isopropyl
^{c}Pr = Cyclopropyl
Bu = ⁿBu = n-Butyl
ⁱBu = Isobutyl
^{t}Bu = t-Butyl
Ph = Phenyl

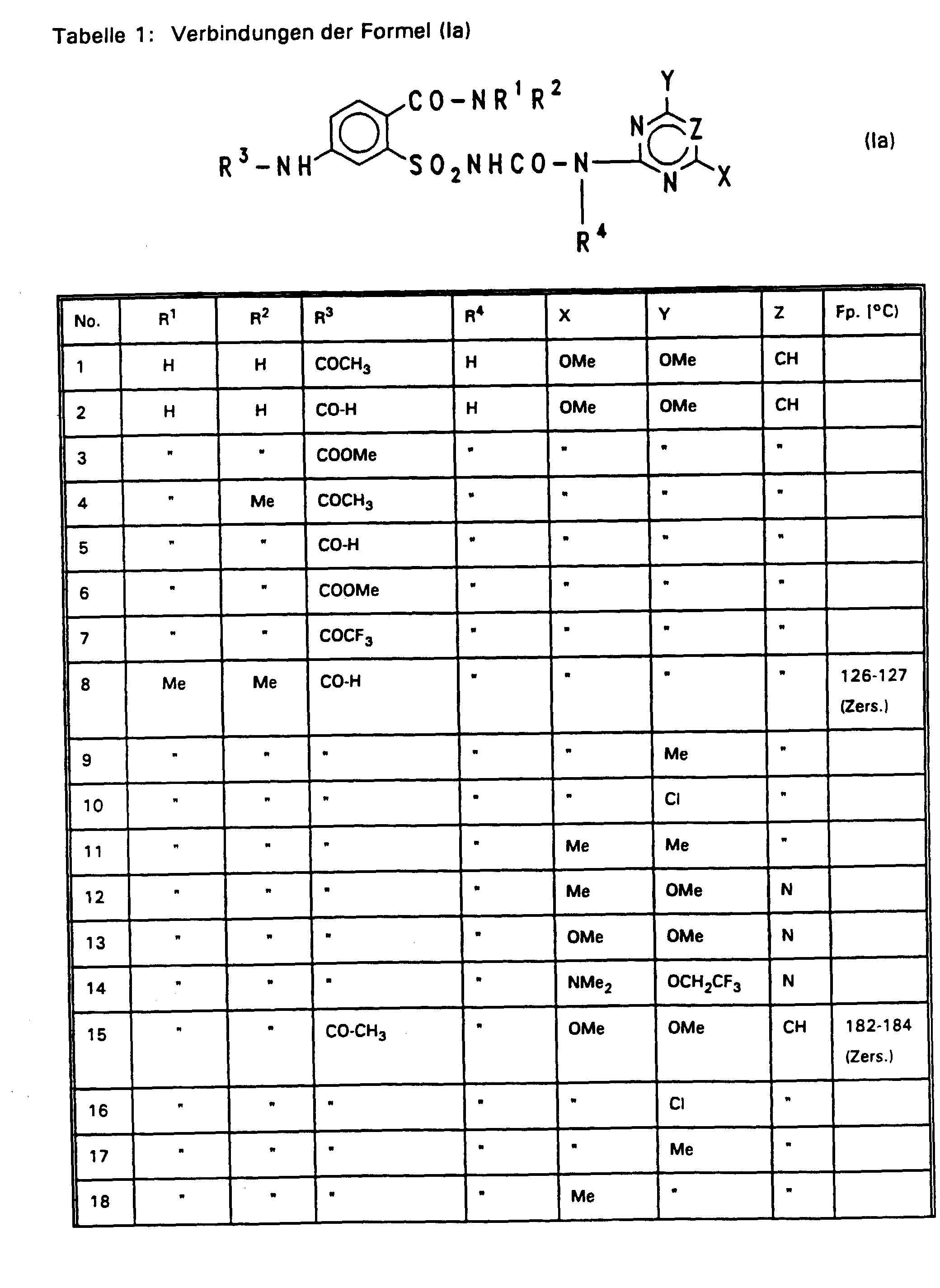

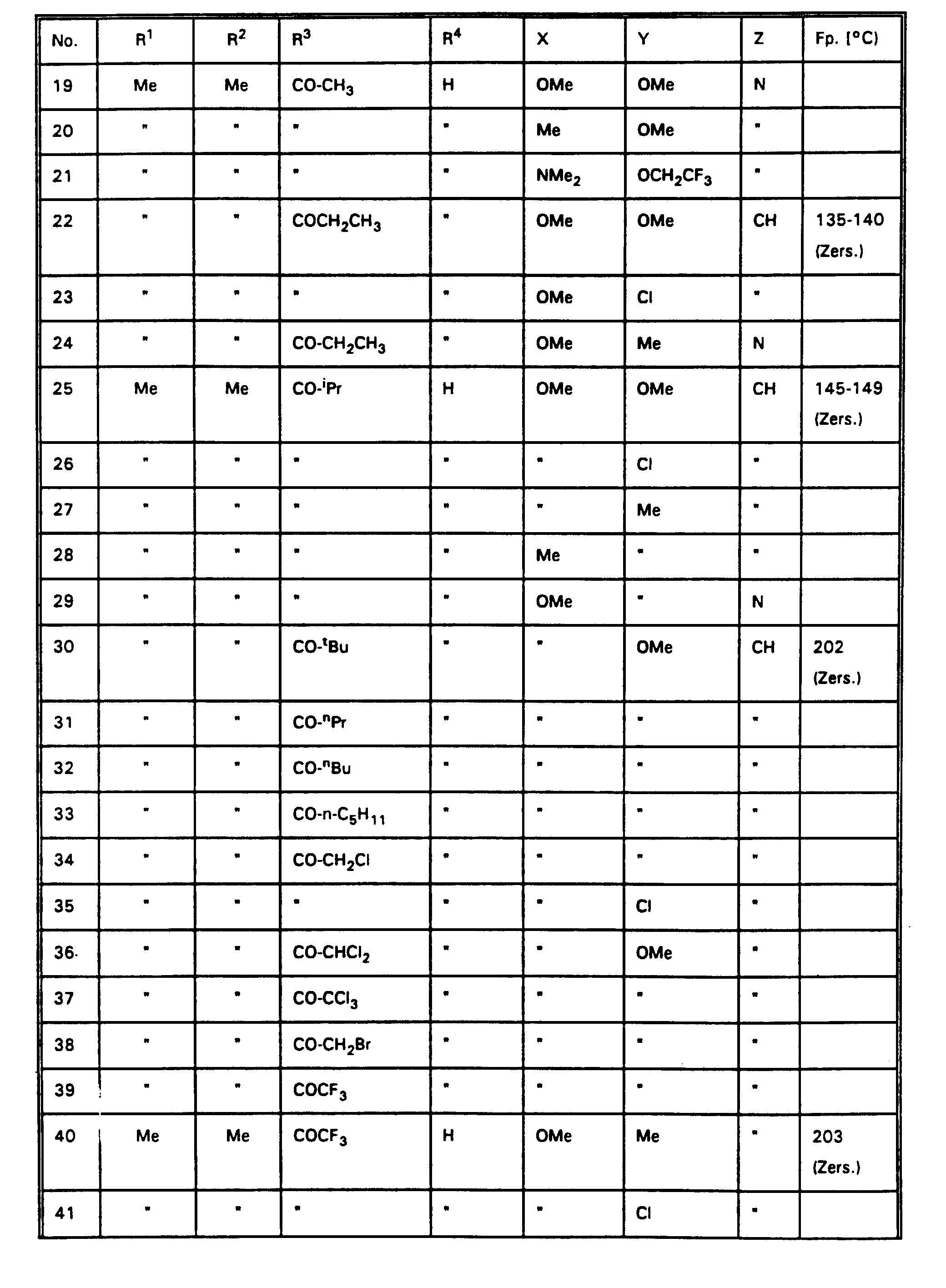

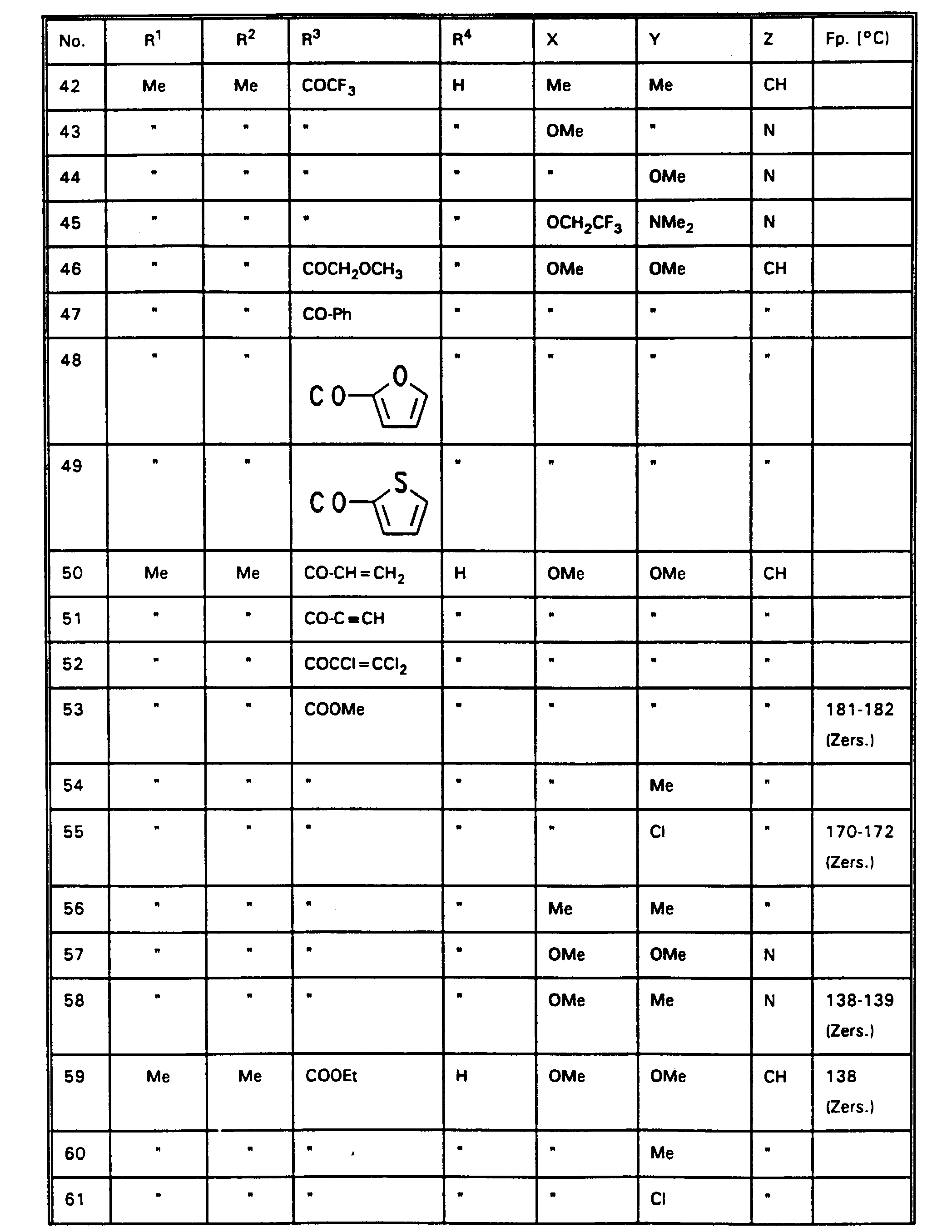

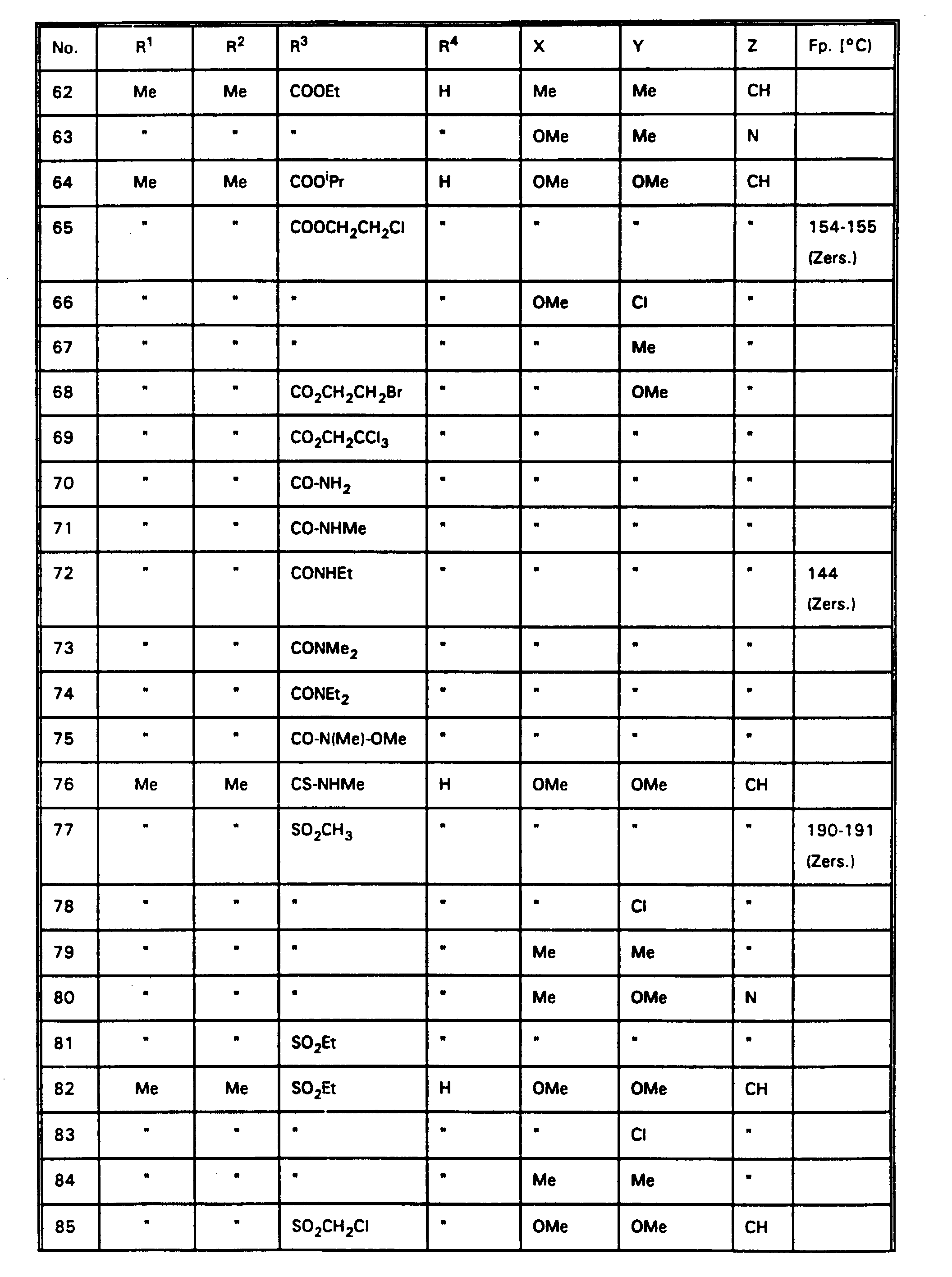

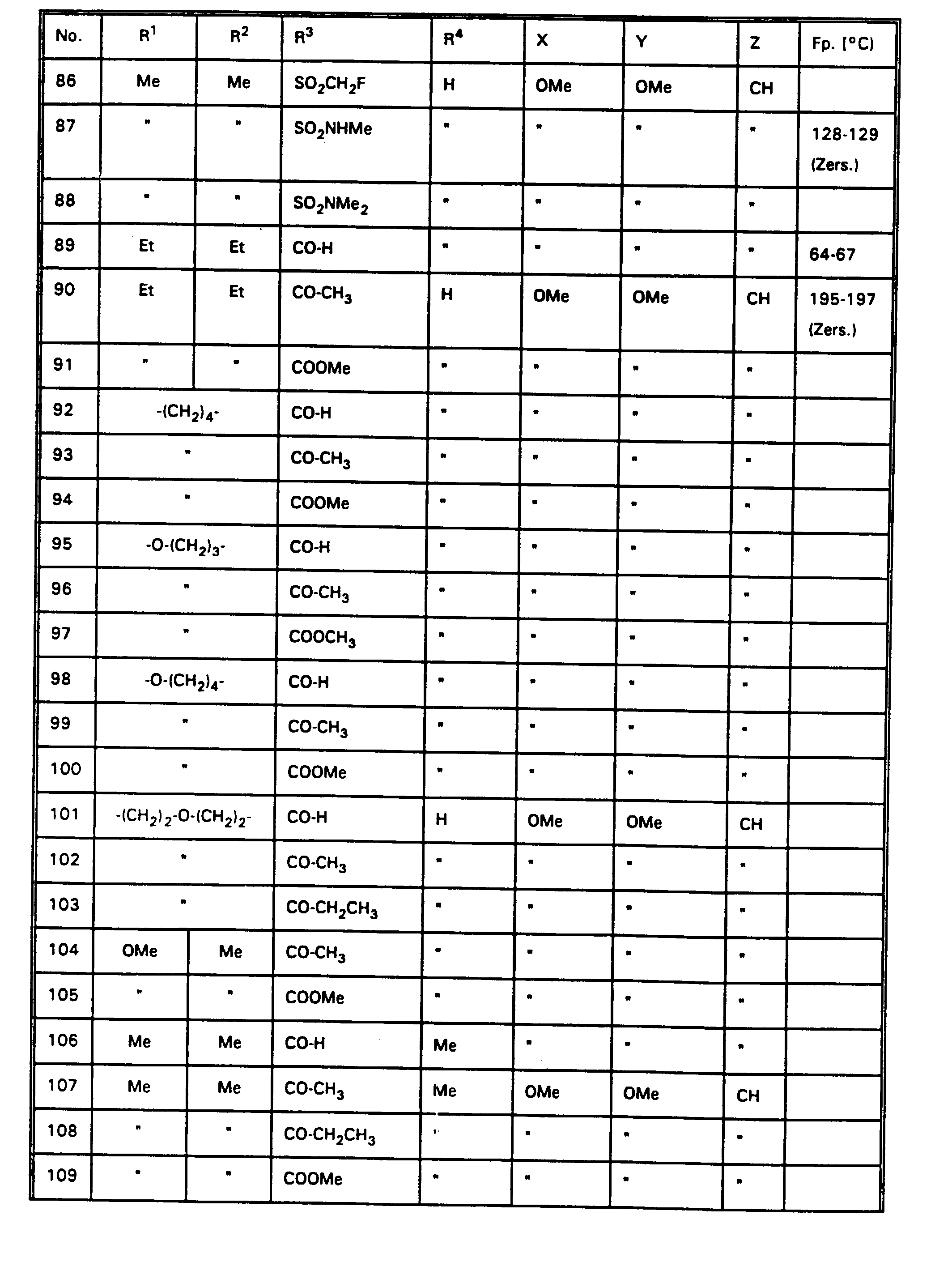

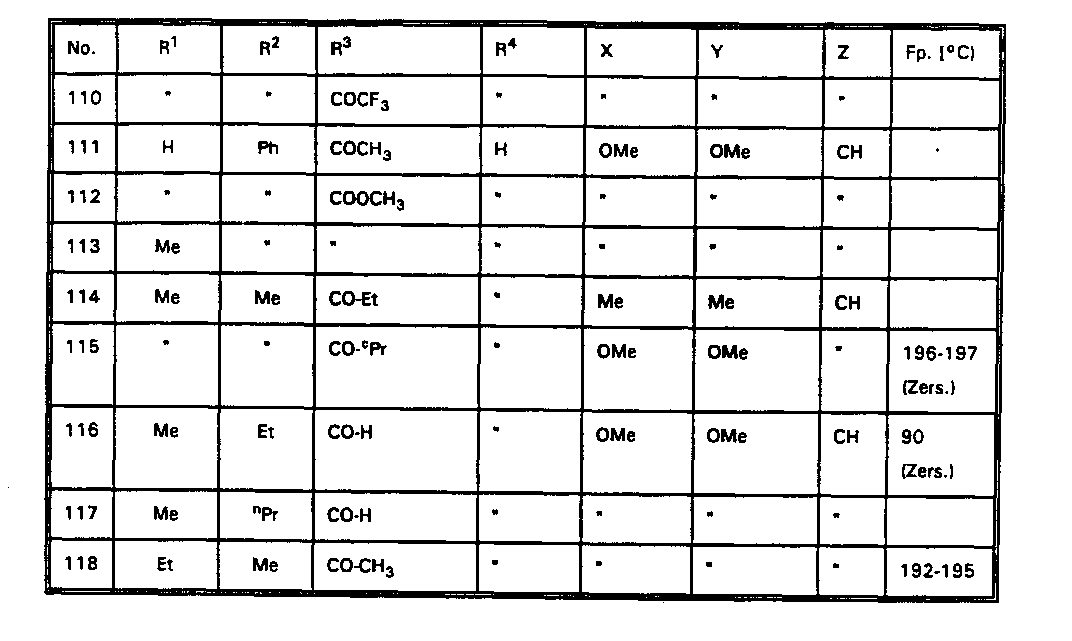

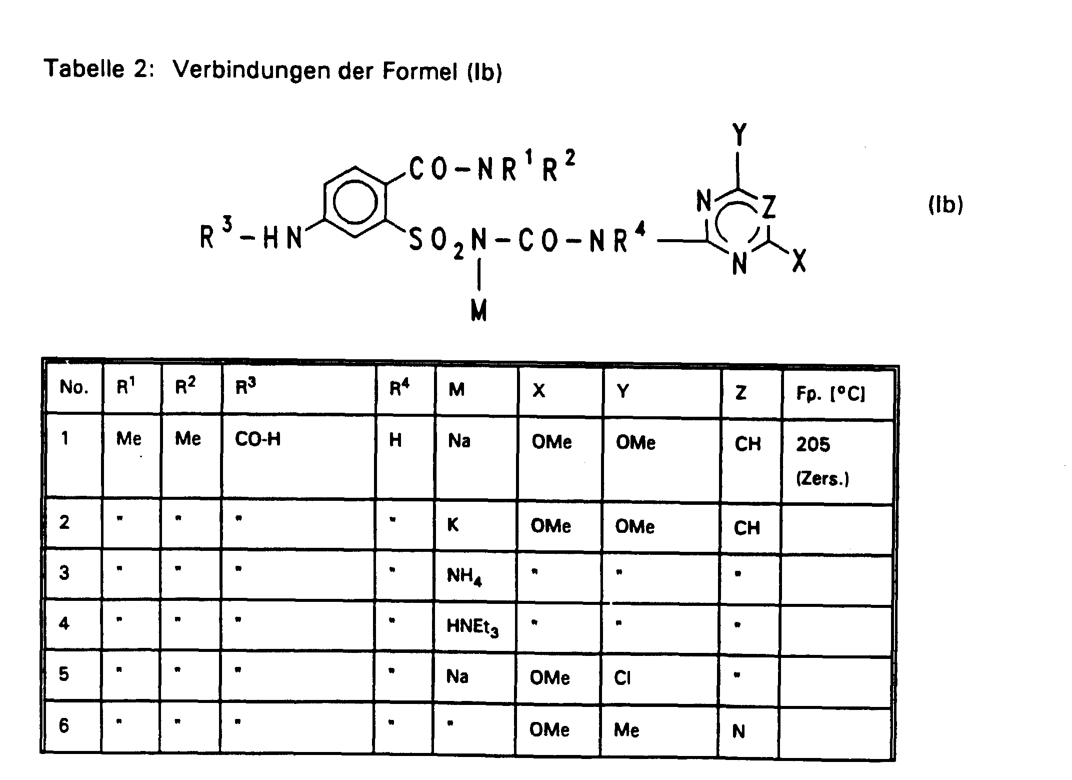

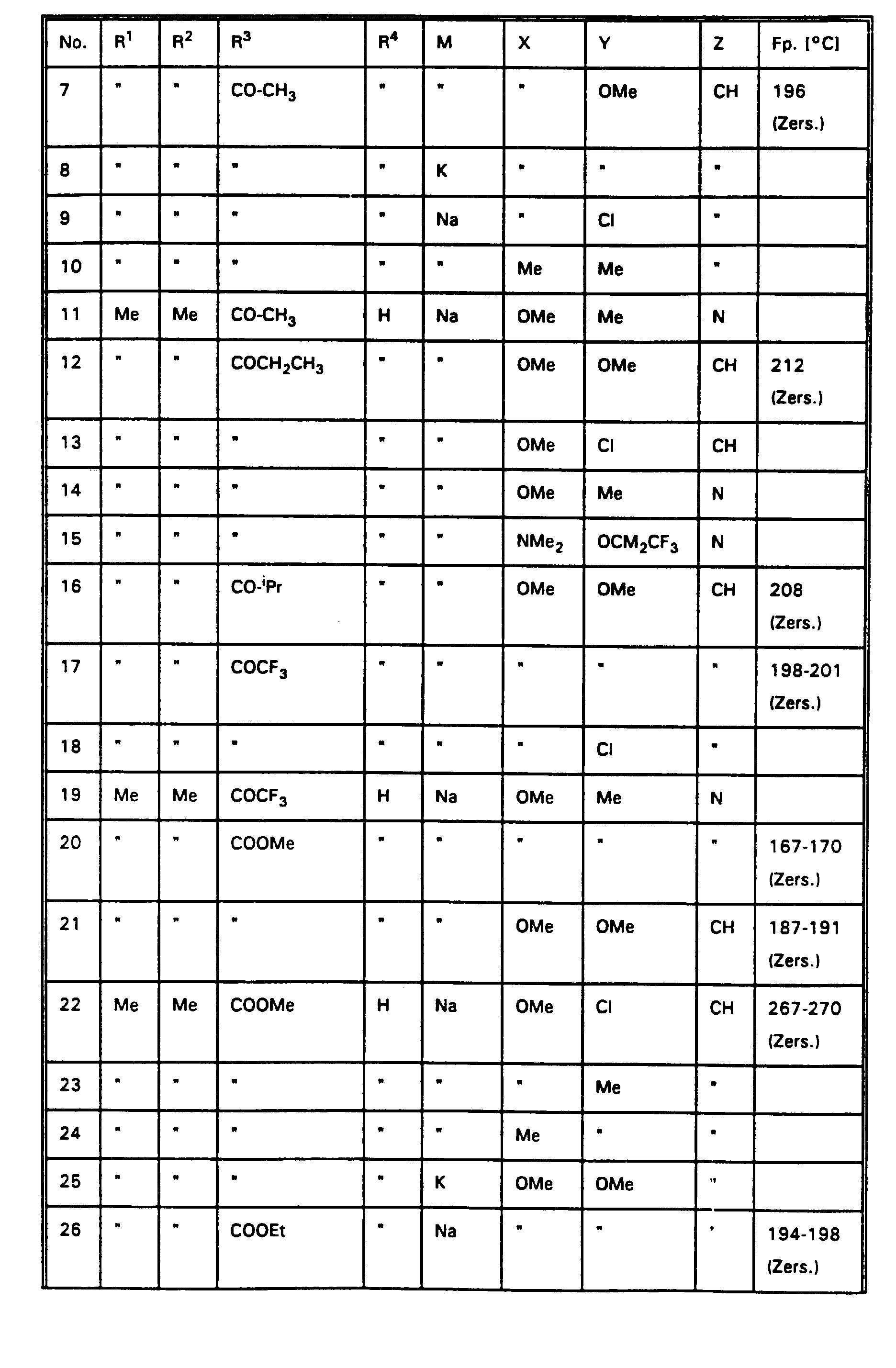

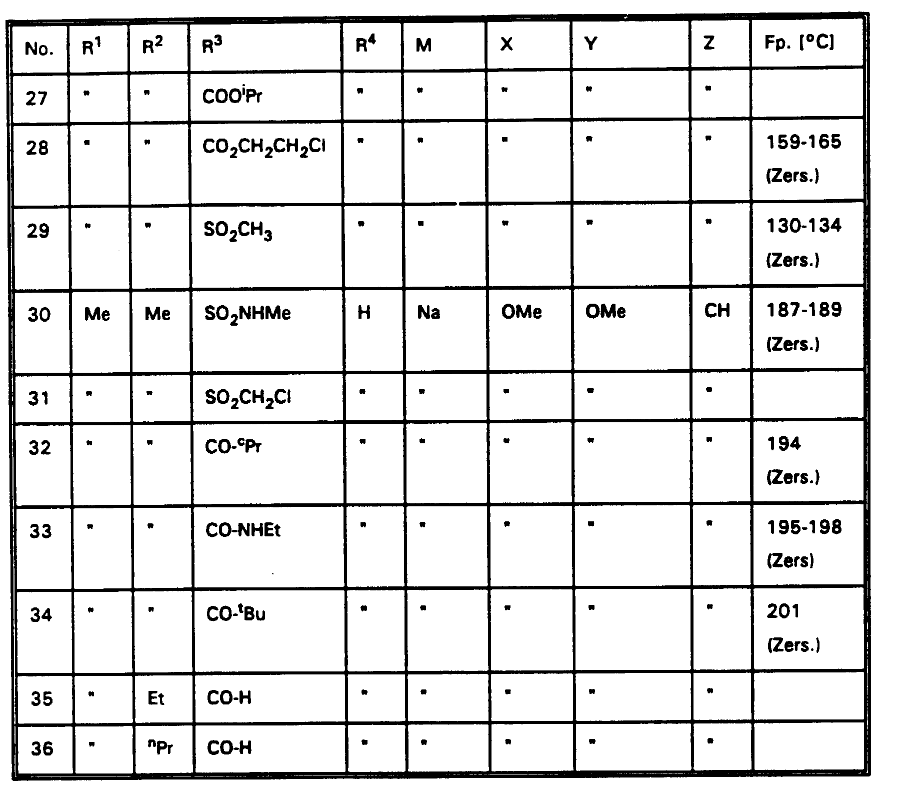

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (1), 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gewichtsteilen Alkylphenolpolyglykolether (®Triton X 207), 3 Gewichtsteilen Isotridecanolpolyglykolether (8 EO) und 71 Gewichtsteilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gewichtsteilen einer Verbindung der Formel (I), 75 Gewichtsteilen Cyclohexanon als Lösungsmittel und 10 Gewichtsteilen oxethyliertes Nonylphenol als Emulgator.
e) Ein in Wasser dispergierbares Granulat wird erhalten indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),
   5 Gewichtsteile 2,2'-dinaphthylmethan-6,6'-disulfonsaures Natrium
   2 Gewichtsteile oleoylmethyltaurinsaures Natrium,
   1 Gewichtsteil Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkraufpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele 8, 15, 22, 25, 30, 40, 44, 53, 55, 58, 59, 65, 72, 77, 87, 115 und 116 aus der Tabelle 1 und die Verbindungen der Beispiele 1, 7, 12, 16, 17, 20, 21, 22, 26, 28-30 und 32-35 aus der Tabelle 2 sehr gute herbizide Wirkung gegen Schadpflanzen wie Sinapis alba, Chrysanthemum segetum, Avena sativa, Stellaria media, Alopecurus myosuroides und Lolium multiflorum im Vorauflaufverfahren bei einer Aufwandmenge von 0,3 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der Beispiele 8, 15, 22, 25, 30, 40, 44, 53, 55, 58, 59, 65, 72, 77, 87, 115 und 116 aus der Tabelle 1 und die Verbindungen der Beispiele 1, 7, 12, 16, 17, 20, 21, 22, 26, 28-30 und 32-35 aus der Tabelle 2 sehr gute herbizide Wirkung gegen Schadpflanzen wie Sinapis alba, Stellaria media, Alopecurus myosuroides, Lolium multiflorum, Chrysanthemum segetum und Avena sativa im Nachauflaufverfahren bei einer Aufwandmenge von 0,3 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt hatten und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschten Pflanzenwuchs in landwirtschaftlichen Kulturen auf.

## Patentansprüche

1. Verbindungen der Formel (I) und deren Salze, 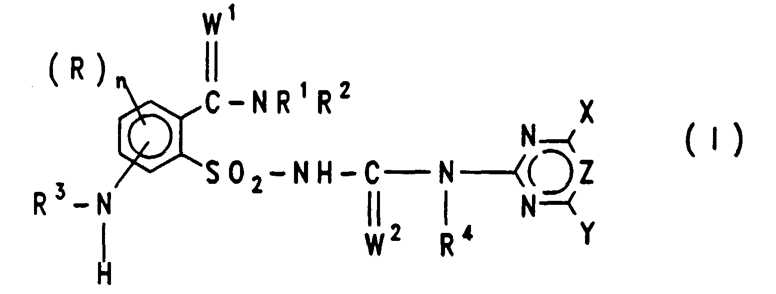 worin
W¹ ein Sauerstoff- oder Schwefelatom,
W² ein Sauerstoff- oder Schwefelatom,
n 0, 1, 2 oder 3,
R Halogen, Alkyl oder Alkoxy, und zwar, wenn n größer 1 ist, unabhängig von anderen Substituenten R,
R¹ Wasserstoff oder einen unsubstituierten oder substituierten Kohlenwasserstoff- oder Kohlenwasserstoffoxyrest,
R² Wasserstoff oder einen unsubstituierten oder substituierten Kohlenwasserstoffrest,
oder die Gruppe
NR¹R² einen heterocyclischen Ring mit 3 bis 8 Ringatomen, der unsubstituiert oder substituiert ist und das N-Atom der Gruppe NR¹R² als Heteroringatom enthält und weitere Heteroringatome enthalten kann,
R³ einen Acylrest,
R⁴ Wasserstoff oder einen aliphatischen Kohlenwasserstoffrest,
X, Y unabhängig voneinander Halogen, C₁-C₆-Alkyl, C₁-C₆-Alkoxy, C₁-C₈-Alkylthio, wobei jeder der drei letztgenannten Reste unsubstituiert oder durch einen oder mehrere Reste aus der Gruppe Halogen, C₁-C₄-Alkoxy und C₁-C₄-Alkylthio substituiert ist, oder C₃-C₆-Cycloalkyl, C₂-C₆-Alkenyl, C₂-C₆-Alkinyl, C₃-C₆-Alkenyloxy oder C₃-C₆-Alkinyloxy und
Z CH oder N bedeuten.

2. Verbindungen und deren Salze nach Anspruch 1, dadurch gekennzeichnet, daß
R¹ H oder Alkyl, Alkenyl, Alkinyl, Alkoxy, Alkenoxy, Alkinoxy, Cycloalkyl, Cycloalkenyl oder Phenyl, wobei jeder der neun letztgenannten Reste unsubstituiert oder substituiert ist und insgesamt bis zu 24 C-Atomen enthält,
R² H oder Alkyl, Alkenyl, Alkinyl, wobei jeder der drei letztgenannten Reste unsubstituiert oder substituiert ist und insgesamt bis zu 24 C-Atomen enthält,
oder die Gruppe
NR¹R² einen unsubstituierten oder substituierten heterocyclischen Ring aus vier bis acht Ringatomen, wobei die Gruppe bis zu insgesamt 18 C-Atomen enthält,
R³ Acyl mit bis zu 24 C-Atomen,
R⁴ H oder Alkyl, Alkenyl oder Alkinyl, wobei jeder der 3 letztgenannten Reste bis zu 12 C-Atomen enthält,
bedeuten.

3. Verbindungen und deren Salze nach Anspruch 1 oder 2, dadurch gekennzeichnet, daß
R¹ H, C₁-C₁₂-Alkyl, C₂-C₁₂-Alkenyl, C₂-C₁₂-Alkinyl, C₁-C₁₂-Alkoxy, C₂-C₁₂-Alkenoxy, C₂-C₁₂-Alkinoxy, C₃-C₈-Cycloalkyl oder C₅-C₈-Cycloalkenyl, wobei jeder der acht letztgenannten Reste unsubstituiert oder durch einen oder mehrere Reste aus der Gruppe Halogen, Cyano, Azido, CO-R⁵, OR⁶, NR⁷R⁸, SR⁹, SO-R¹⁰ und SO₂-R¹¹ substituiert ist, oder unsubstituiertes oder substituiertes Phenyl,
R² H, C₁-C₁₂-Alkyl, C₂-C₁₂-Alkenyl oder C₂-C₁₂-Alkinyl, wobei jeder der drei letztgenannten Reste unsubstituiert oder durch einen oder mehrere Reste aus der Gruppe Halogen, Cyano, Azido, COR¹², OR¹³, NR¹⁴R¹⁵, SR¹⁶, SO-R¹⁷ und SO₂-R¹⁸ substituiert ist,
oder die Gruppe
NR¹R² einen heterocyclischen Ring aus vier bis acht Ringatomen, der bis zu zwei weitere Heteroringatome aus der Gruppe N, O und S im Ring enthalten kann und der unsubstituiert oder durch einen oder mehrere Reste aus der Gruppe Halogen, C₁-C₆-Alkyl, NO₂, N₃ und CN substituiert ist,
R³ CO-R¹⁹, CS-R²⁰, SO₂-R²¹, SO-R²¹ oder C(=NR²¹)-R¹⁹,
R⁴ H, C₁-C₃-Alkyl, C₂-C₅-Alkenyl oder C₂-C₅-Alkinyl,
R⁵ H, C₁-C₅-Alkyl, C₂-C₅-Alkenyl, C₂-C₅-Alkinyl, C₁-C₅-Alkoxy, C₂-C₅-Alkenoxy, C₂-C₅-Alkinoxy, NR²²R²³ oder OH,
R⁶ H, C₁-C₅-Alkyl, C₁-C₅-Haloalkyl, C₂-C₅-Alkenyl, C₂-C₅-Haloalkenyl, C₂-C₅-Alkinyl, C₂-C₅-Haloalkinyl,
R⁷ H, C₁-C₅-Alkyl, C₂-C₅-Alkenyl, C₂-C₅-Alkinyl, C₁-C₅-Alkoxy, CO-CH₃, CO-H, COOCH₃,
R⁸ H, C₁-C₅-Alkyl, C₂-C₅-Alkenyl, C₂-C₅-Alkinyl
oder die Gruppe
NR⁷R⁸ einen heterocyclischen Ring aus vier bis acht Ringatomen, der bis zu zwei weitere Heteroringatome aus der Gruppe N, O und S im Ring enthalten kann und der unsubstituiert oder durch einen oder mehrere Reste aus der Gruppe Halogen, C₁-C₆-Alkyl, NO₂, N₃ und CN substituiert ist,
R⁹ H, C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl,
R¹⁰ C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl,
R¹¹ C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl, C₁-C₅-Alkoxyalkyl, C₃-C₈-Cycloalkyl oder C₅-C₈-Cycloalkenyl,
R¹² einen Rest analog R⁵,
R¹³ einen Rest analog R⁶,
R¹⁴ einen Rest analog R⁷,
R¹⁵ einen Rest analog R⁸
oder die Gruppe
NR¹⁴R¹⁵ eine Gruppe analog NR⁷R⁸,
R¹⁶ einen Rest analog R⁹,
R¹⁷ einen Rest analog R¹⁰,
R¹⁸ einen Rest analog R¹¹,
R¹⁹ H, C₁-C₁₂-Alkyl, C₂-C₁₂-Alkenyl, C₂-C₁₂-Alkinyl, C₁-C₁₂-Alkoxy, C₁-C₁₂-Alkylthio, C₂-C₁₂-Alkenoxy, C₂-C₁₂-Alkinoxy, C₃-C₈-Cycloalkyl, C₅-C₈-Cycloalkenyl, C₁-C₁₂-Alkylamino, Di-(C₁-C₆-Alkyl)-amino, N-C₁-C₄-Alkoxy-N-C₁-C₄-alkyl-amino, wobei jeder der 13 letztgenannten Reste unsubstituiert oder durch einen oder mehrere Reste aus der Gruppe Halogen, Cyano, Azido, CO-R²⁴, OR²⁵, NR²⁶R²⁷, SR²⁸, SOR²⁹ und SO₂R³⁰ substituiert ist, oder einen unsubstituierten oder substituierten Phenyl-, Phenoxy- oder Phenylaminorest,
R²⁰ einen Rest analog R¹⁹,
R²¹ einen Rest analog R¹¹,
R²² H, C₁-C₅-Alkyl, C₂-C₅-Alkenyl, C₂-C₅-Alkinyl, C₁-C₅-Alkoxy, C₂-C₅-Alkenoxy, C₂-C₅-Alkinoxy, wobei jeder der letztgenannten Reste unsubstituiert oder durch einen oder mehrere Reste aus der Gruppe Halogen, Cyano, Azido, Amino, mono- und disubstituiertes Amino, C₁-C₃-Alkyl, C₁-C₃-Alkoxy, C₁-C₃-Alkylsulfonyl, C₁-C₃-Alkylsulfinyl und C₁-C₃-Alkylthio substituiert ist,
R²³ H, C₁-C₅-Alkyl, C₂-C₅-Alkenyl, C₂-C₅-Alkinyl, C₁-C₅-Alkoxy, C₂-C₅-Alkenoxy, wobei jeder der 5 letztgenannten Reste unsubstituiert oder durch einen oder mehrere Reste aus der Gruppe Halogen, Cyano, Azido, Amino, mono- und disubstituiertes Amino, C₁-C₃-Alkoxy, C₁-C₃-Alkylthio, C₁-C₃-Alkylsulfonyl und C₁-C₃-Alkylsulfinyl substituiert ist,
oder die Gruppe
NR²²R²³ einen heterocyclischen Ring analog NR⁷R⁸,
R²⁴ H, C₁-C₃-Alkyl, C₁-C₃-Alkoxy, NH₂, mono- bzw. disubstituiertes Amino,
R²⁵ H, C₁-C₅-Alkyl, C₂-C₅-Alkenyl, C₂-C₅-Alkinyl, C₁-C₅-Haloalkyl, C₂-C₅-Haloalkenyl, C₂-C₅-Haloalkinyl,
R²⁶ H, C₁-C₃-Alkyl, C₁-C₃-Alkoxy, CO-CH₃, CO-H,
R²⁷ H, C₁-C₃-Alkyl
oder die Gruppe
NR²⁶R²⁷ eine Gruppe analog NR²²R²³,
R²⁸ H, C₁-C₃-Alkyl, C₁-C₃-Haloalkyl, C₂-C₅-Alkenyl, C₂-C₅-Haloalkenyl, C₂-C₅-Alkinyl, C₂-C₅-Haloalkinyl,
R²⁹ C₁-C₅-Alkyl, C₁-C₅-Haloalkyl, C₂-C₅-Alkenyl, C₂-C₅-Haloalkenyl, C₂-C₅-Alkinyl, C₂-C₅-Haloalkinyl,
R³⁰ analog R²⁹ und
X, Y unabhängig voneinander Halogen, C₁-C₄-Alkyl, C₁-C₄-Alkoxy, C₁-C₄-Alkylthio, wobei jeder der drei letztgenannten Reste unsubstituiert oder durch einen oder mehrere Reste aus der Gruppe Halogen, C₁-C₄-Alkoxy und C₁-C₄-Alkylthio substituiert ist,
Mono- oder Di(C₁-C₄-alkyl)amino, C₃-C₆-Cycloalkyl, C₃-C₅-Alkenyl oder C₃-C₅-Alkinyloxy
bedeuten.

4. Verbindungen und deren Salze nach einem der Ansprüche 1 bis 3, dadurch gekennzeichnet, daß die Gruppe NHR³ am Phenylring in meta-Stellung zur SO₂-Gruppe und in para-Stellung zur Gruppe CW¹-NR¹R² steht und daß
W¹ ein Sauerstoffatom,
W² ein Sauerstoffatom,
n 0,
R¹ H, C₁-C₂-Alkyl, C₁-C₂-Alkoxy, Phenyl, das unsubstituiert oder durch einen oder mehrere Reste aus der Gruppe Halogen, C₁-C₂-Alkyl, C₁-C₂-Alkoxy, C₁-C₂-Halogenalkyl und C₁-C₂-Halogenalkoxy substituiert ist,
R² H, C₁-C₂-Alkyl oder C₁-C₂-Alkoxy
oder die Gruppe
NR¹R² einen heterocyclischen Ring aus 5 oder 6 Ringatomen, der bis zu einem weiteren Heteroringatom aus der Gruppe N und O im Ring enthalten kann und der unsubstituiert oder durch einen oder mehrere Reste aus der Gruppe C₁-C₂-Alkyl substituiert ist,
R³ CO-R¹⁹, CS-R²⁰ oder SO₂-R²¹,
R⁴ H oder CH₃,
R¹⁹ H, C₁-C₄-Alkyl, C₂-C₄-Alkenyl, C₂-C₄-Alkinyl, C₁-C₄-Alkoxy, C₁-C₄-Alkylthio, C₂-C₄-Alkenoxy, C₂-C₄-Alkinoxy, C₃-C₆-Cycloalkyl, C₁-C₄-Alkylamino, Di-(C₁-C₄-Alkyl)-amino, N-C₁-C₂-Alkoxy-N-C₁-C₂-alkyl-amino, wobei jeder der 10 letztgenannten Reste unsubstituiert oder durch einen oder mehrere Reste aus der Gruppe Halogen und C₁-C₄-Alkoxy substituiert ist,
oder einen Phenyl- oder Phenoxyrest, der unsubstituiert oder durch Reste aus der Gruppe Halogen, C₁-C₂-Alkyl, C₁-C₂-Alkoxy, C₁-C₂-Halogenalkyl und C₁-C₂-Halogenalkoxy substituiert ist,
R²⁰ einen Rest analog R¹⁹ und
R²¹ C₁-C₃-Alkyl, C₁-C₃-Haloalkyl, C₁-C₃-Alkoxy-C₁-C₂-alkyl,
einer der Reste X und Y
Halogen, C₁-C₂-Alkyl, C₁-C₂-Alkoxy, C₁-C₂-Alkylthio, wobei jeder der drei letztgenannten Reste unsubstituiert oder durch einen oder mehrere Reste aus der Gruppe Halogen, C₁-C₂-Alkoxy und C₁-C₂-Alkylthio substituiert ist, oder Mono- oder Di(C₁-C₂-alkyl)amino und
der andere der Reste X und Y
C₁-C₂-Alkyl, C₁-C₂-Haloalkyl, C₁-C₂-Alkoxy, C₁-C₂-Haloalkoxy oder C₁-C₂-Alkylthio und
Z CH oder N
bedeuten.

5. Verfahren zur Herstellung der Verbindungen der Formel (I) oder deren Salze nach einem der Ansprüche 1 bis 4, dadurch gekennzeichnet, daß man
a) eine Verbindung der Formel (II) 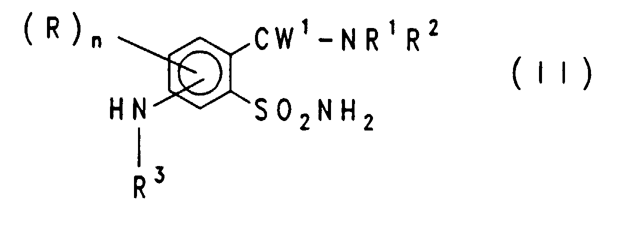 mit einem heterocyclischen Carbamat der Formel (III), 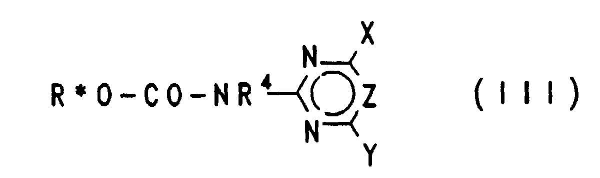 worin
R* unsubstituiertes oder substituiertes Phenyl oder C₁-C₄-Alkyl bedeutet, umsetzt oder
b) ein Sulfonylisocyanat der Formel (IV) 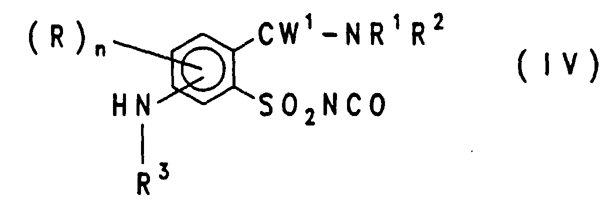 mit einem heterocyclischen Amin der Formel (V) 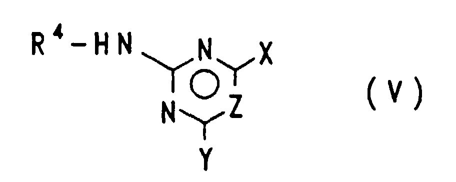 umsetzt oder
c) ein Sulfochlorid der Formel (VI) 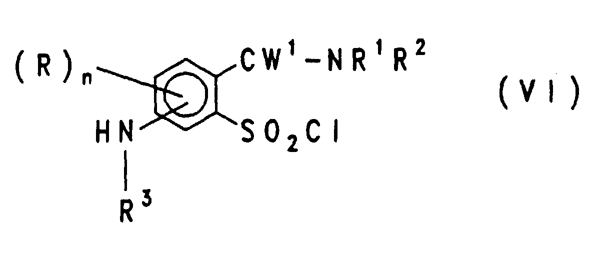 mit einem heterocyclischen Amin der genannten Formel (V) in Gegenwart eines Cyanats umsetzt oder
d) ein Sulfonamid der genannten Formel (II) mit einem (Thio-)lsocyanat der Formel (VII) 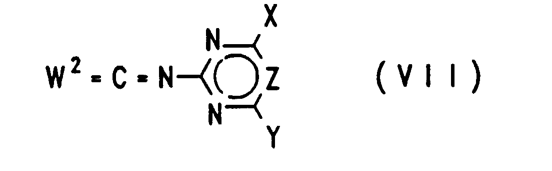 in Gegenwart einer geeigneten Base, wie z.B. Kaliumcarbonat oder Triethylamin, umsetzt,
wobei in den obigen Formeln (II) bis (VII) die Reste R, R¹, R², R³, R⁴, W¹, W², X, Y und Z sowie der Index n wie in Formel (I) definiert sind und wobei in den Varianten a) - c) zunächst Verbindungen der Formel (I) erhalten werden, in denen W² ein Sauerstoffatom bedeutet.

6. Herbizides oder pflanzenwachstumsregulierendes Mittel, dadurch gekennzeichnet, daß es eine wirksame Menge mindestens einer Verbindung der Formel (I) oder eines ihrer Salze nach einem der Ansprüche 1 bis 4 und im Pflanzenschutz übliche Formulierungshilfsmittel enthält.

7. Verfahren zur Bekämpfung von Schadpflanzen oder zur Wachstumsregulierung von Pflanzen, dadurch gekennzeichnet, daß man eine wirksame Menge mindestens einer Verbindung der Formel (I) oder eines ihrer Salze nach einem der Ansprüche 1 bis 4 auf die Schadpflanzen bzw. Pflanzen, deren Pflanzensamen oder die Fläche, auf der die Pflanzen wachsen, appliziert.

8. Verwendung der Verbindungen der Formel (I) oder deren Salze nach einem der Ansprüche 1 bis 4 als Herbizide oder Pflanzenwachstumsregulatoren.

9. Verbindungen der Formeln (II), (IV), (VI), wie sie in Anspruch 5 definiert sind.

10. Verbindungen der Formel (VIII)* 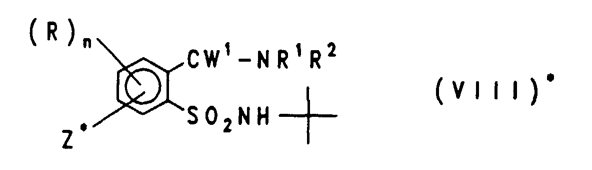
Z* = NHR³ (VIII)
Z* = NH₂ (IX)
Z* = NO₂ (X)
worin
Z* NHR³, NH₂ oder NO₂ bedeutet und
R, n, R¹, R², R³ und W¹ wie in Formel (I) nach Anspruch 1 definiert sind.

## Claims

1. A compound of the formula (I) or a salt thereof 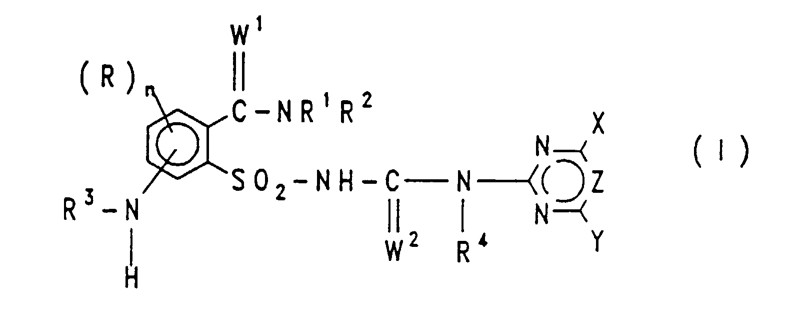 in which
W¹ is an oxygen or sulfur atom,
W² is an oxygen or sulfur atom,
n is 0, 1, 2 or 3,
R is halogen, alkyl or alkoxy, independently of other substituents R if n is greater than 1,
R¹ is hydrogen or an unsubstituted or substituted hydrocarbon or hydrocarbonoxy radical,
R² is hydrogen or an unsubstituted or substituted hydrocarbon radical,
or the group
NR¹R² is a heterocyclic ring having 3 to 8 ring atoms, which is unsubstituted or substituted and contains the N atom of the group NR¹R² as a hetero-ring atom and can contain further hetero-ring atoms,
R³ is an acyl radical,
R⁴ is hydrogen or an aliphatic hydrocarbon radical,
X, Y independently of one another are halogen, C₁-C₆-alkyl, C₁-C₆-alkoxy, C₁-C₆-alkylthio, where each of the three radicals mentioned last is unsubstituted or substituted by one or more radicals from the group consisting of halogen, C₁-C₄-alkoxy and C₁-C₄alkylthio, or C₃-C₆-cycloalkyl, C₂-C₆-alkenyl, C₂-C₆-alkynyl, C₃-C₆-alkenyloxy or C₃-C₆-alkyqyloxy, and
Z is CH or N.

2. A compound or a salt thereof as claimed in claim 1, wherein
R¹ is H or alkyl, alkenyl, alkynyl, alkoxy, alkenoxy, alkynoxy, cycloalkyl, cycloalkenyl or phenyl, where each of the nine radicals mentioned last is unsubstituted or substituted and contains a total of up to 24 carbon atoms,
R² is H or alkyl, alkenyl or alkynyl, where each of the three radicals mentioned last is unsubstituted or substituted and contains a total of up to 24 carbon atoms,
or the group
NR¹R² is an unsubstituted or substituted heterocyclic ring of four to eight ring atoms, where the group contains up to a total of 18 carbon atoms,
R³ is acyl having up to 24 carbon atoms, and
R⁴ is H or alkyl, alkenyl or alkynyl, where each of the 3 radicals mentioned last contains up to 12 carbon atoms.

3. A compound or a salt thereof as claimed in claim 1 or 2, wherein
R¹ is H, C₁-C₁₂-alkyl, C₂-C₁₂-alkenyl, C₂-C₁₂-alkynyl, C₁-C₁₂-alkoxy, C₂-C₁₂-alkenoxy, C₂-C₁₂-alkynoxy, C₃-C₈-cycloalkyl or C₅-C₈-cycloalkenyl, where each of the eight radicals mentioned last is unsubstituted or substituted by one or more radicals from the group consisting of halogen, cyano, azido, CO-R⁵, OR⁶, NR⁷R⁸, SR⁹, SO-R¹⁰ and SO₂-R¹¹, or unsubstituted or substituted phenyl,
R² is H, C₁-C₁₂-alkyl, C₂-C₁₂-alkenyl or C₂-C₁₂-alkynyl, where each of the three radicals mentioned last is unsubstituted or substituted by one or more radicals from the group consisting of halogen, cyano, azido, COR¹², OR¹³, NR¹⁴R¹⁵, SR¹⁶, SO-R¹⁷ and SO₂-R¹⁸,
or the group
NR¹R² is a heterocyclic ring of four to eight ring atoms, which can contain up to two further hetero-ring atoms in the ring from the group consisting of N, O and S and is unsubstituted or substituted by one or more radicals from the group consisting of halogen, C₁-C₆-alkyl, NO₂, N₃ and CN,
R³ is CO-R¹⁹, CS-R²⁰, SO₂-R²¹, SO-R²¹ or C(=NR²¹)-R¹⁹,
R⁴ is H, C₁-C₃-alkyl, C₂-C₅-alkenyl or C₂-C₅-alkynyl,
R⁵ is H, C₁-C₅-alkyl, C₂-C₅-alkenyl, C₂-C₅-alkynyl, C₁-C₅-alkoxy, C₂-C₅-alkenoxy, C₂-C₅-alkynoxy, NR²²R²³ or OH,
R⁶ is H, C₁-C₅-alkyl, C₁-C₅-haloalkyl, C₂-C₅-alkenyl, C₂-C₅-haloalkenyl, C₂-C₅-alkynyl or C₁-C₅-haloalkynyl,
R⁷ is H, C₁-C₅-alkyl, C₂-C₅-alkenyl, C₂-C₅-alkynyl, C₁-C₅-alkoxy, CO-CH₃, CO-H or COOCH₃,
R⁸ is H, C₁-C₅-alkyl, C₂-C₅-alkenyl or C₂-C₅-alkynyl
or the group
NR⁷R⁸ is a heterocyclic ring of four to eight ring atoms, which can contain up to two further hetero-ring atoms in the ring from the group consisting of N, O and S and is unsubstituted or substituted by one or more radicals from the group consisting of halogen, C₁-C₆-alkyl, NO₂, N₃ and CN,
R⁹ is H, C₁-C₅-alkyl, C₂-C₅-alkenyl, C₂-C₅-alkynyl, C₁-C₅-haloalkyl, C₂-C₅-haloalkenyl or C₂-C₅-haloalkynyl,
R¹⁰ is C₁-C₅-alkyl, C₂-C₅-alkenyl, C₂-C₅-alkynyl, C₁-C₅-haloalkyl, C₂-C₅-haloalkenyl or C₂-C₅-haloalkynyl,
R¹¹ is C₁-C₅-alkyl, C₂-C₅-alkenyl, C₂-C₅-alkynyl, C₁-C₅-haloalkyl, C₂-C₅-haloalkenyl, C₂-C₅-haloalkynyl, C₁-C₅-alkoxyalkyl, C₃-C₈-cycloalkyl or C₅-C₈-cycloalkenyl,
R¹² is a radical analogous to R⁵,
R¹³ is a radical analogous to R⁶,
R¹⁴ is a radical analogous to R⁷,
R¹⁵ is a radical analogous to R⁸
or the group
NR¹⁴R¹⁵ is a group analogous to NR⁷R⁸,
R¹⁶ is a radical analogous to R⁹,
R¹⁷ is a radical analogous to R¹⁰,
R¹⁸ is a radical analogous to R¹¹,
R¹⁹ is H, C₁-C₁₂-alkyl, C₂-C₁₂-alkenyl, C₂-C₁₂-alkynyl, C₁-C₁₂-alkoxy, C₁-C₁₂-alkylthio, C₂-C₁₂-alkenoxy, C₂-C₁₂-alkynoxy, C₃-C₈-cycloalkyl, C₅-C₈-cycloalkenyl, C₁-C₁₂-alkylamino, di(C₁-C₆-alkyl)amino, N-C₁-C₄-alkoxy-N-C₁-C₄-alkylamino, where each of the 13 radicals mentioned last are unsubstituted or substituted by one or more radicals from the group consisting of halogen, cyano, azido, CO-R²⁴, OR²⁵, NR²⁶R²⁷, SR²⁸, SOR²⁹ and SO₂R³⁰, or an unsubstituted or substituted phenyl, phenoxy or phenylamino radical,
R²⁰ is a radical analogous to R¹⁹,
R²¹ is a radical analogous to R¹¹,
R²² is H, C₁-C₅-alkyl, C₂-C₅-alkenyl, C₂-C₅-alkynyl, C₁-C₅-alkoxy, C₂-C₅-alkenoxy or C₂-C₅-alkynoxy, where each of the radicals mentioned last is unsubstituted or substituted by one or more radicals from the group consisting of halogen, cyano, azido, amino, mono- and disubstituted amino, C₁-C₃-alkyl, C₁-C₃-alkoxy, C₁-C₃-alkylsulfonyl, C₁-C₃-alkylsulfinyl and C₁-C₃-alkylthio,
R²³ is H, C₁-C₅-alkyl, C₂-C₅-alkenyl, C₂-C₅-alkynyl, C₁-C₅-alkoxy or C₂-C₅-alkenoxy, where each of the 5 radicals mentioned last is unsubstituted or substituted by one or more radicals from the group consisting of halogen, cyano, azido, amino, mono- and disubstituted amino, C₁-C₃-alkoxy, C₁-C₃-alkylthio, C₁-C₃-alkylsulfonyl and C₁-C₃-alkylsulfinyl,
or the group
NR²²R²³ is a heterocyclic ring analogous to NR⁷R⁸,
R²⁴ is H, C₁-C₃-alkyl, C₁-C₃-alkoxy, NH₂ or mono- or disubstituted amino,
R²⁵ is H, C₁-C₅-alkyl, C₂-C₅-alkenyl, C₂-C₅-alkynyl, C₁-C₅-haloalkyl, C₂-C₅-haloalkenyl or C₂-C₅-haloalkynyl,
R²⁶ is H, C₁-C₃-alkyl, C₁-C₃-alkoxy, CO-CH₃ or CO-H,
R²⁷ is H or C₁-C₃-alkyl
or the group
NR²⁶R²⁷ is a group analogous to NR²²R²³,
R²⁸ is H, C₁-C₃-a1kyl, C₁-C₃-haloalkyl, C₂-C₅-alkenyl, C₂-C₅-haloalkenyl, C₂-C₅-alkynyl or C₁-C₅-haloalkynyl,
R²⁹ is C₁-C₅-alkyl, C₁-C₅-haloalkyl, C₂-C₅-alkenyl, C₂-C₅-haloalkenyl, C₂-C₅-alkynyl or C₂-C₅-haloalkynyl,
R³⁰ is analogous to R²⁹, and
X, Y independently of one another are halogen, C₁-C₄-alkyl, C₁-C₄-alkoxy or C₁-C₄-alkylthio, where each of the three radicals mentioned last is unsubstituted or substituted by one or more radicals from the group consisting of halogen, C₁-C₄-alkoxy and C₁-C₄-alkylthio, or mono- or di (C₁-C₄-alkyl ) amino, C₃-C₆-cycloalkyl, C₃-C₅-alkenyl or C₃-C₅-alkynyloxy.

4. A compound or a salt thereof as claimed in one of claims 1 to 3, wherein the group NHR³ on the phenyl ring is in the meta-position relative to the SO₂ group and in the para-position relative to the group CW¹-NR¹R², and wherein
W¹ is an oxygen atom,
W² is an oxygen atom,
n is 0,
R¹ is H, C₁-C₂-alkyl, C₁-C₂-alkoxy or phenyl which is unsubstituted or substituted by one or more radicals from the group consisting of halogen, C₁-C₂-alkyl, C₁-C₂-alkoxy, C₁-C₂-haloalkyl and C₁-C₂-haloalkoxy,
R² is H, C₁-C₂-alkyl or C₁-C₂-alkoxy
or the group
NR¹R² is a heterocyclic ring of 5 or 6 ring atoms, which can contain up to one further hetero-ring atom in the ring from the group consisting of N and O and is unsubstituted or substituted by one or more radicals from the group consisting of C₁-C₂-alkyl,
R³ is CO-R¹⁹, CS-R²⁰ or SO₂-R²¹,
R⁴ is H or CH₃,
R¹⁹ is H, C₁-C₄-alkyl, C₂-C₄-alkenyl, C₂-C₄-alkynyl, C₁-C₄-alkoxy, C₁-C₄-alkylthio, C₂-C₄-alkenoxy, C₂-C₄-alkynoxy, C₃-C₆-cycloalkyl, C₁-C₄-alkylamino, di-(C₁-C₄-alkyl)amino, N-C₁-C₂-alkoxy-N-C₁-C₂-alkyl-amino, where each of the 10 radicals mentioned last is unsubstituted or substituted by one or more radicals from the group consisting of halogen and C₁-C₄-alkoxy, or a phenyl or phenoxy radical, which is unsubstituted or substituted by radicals from the group consisting of halogen, C₁-C₂-alkyl, C₁-C₂-alkoxy, C₁-C₂-haloalkyl and C₁-C₂-haloalkoxy,
R²⁰ is a radical analogous to R¹⁹, and
R²¹ is C₁-C₃-alkyl, C₁-C₃-haloalkyl or C₁-C₃-alkoxyC₁-C₂-alkyl,
one of the radicals X and Y is
halogen, C₁-C₂-alkyl, C₁-C₂-alkoxy, C₁-C₂-alkylthio where each of the three radicals mentioned last is unsubstituted or substituted by one or more radicals from the group consisting of halogen, C₁-C₂-alkoxy and C₁-C₂-alkylthio, or mono- or di(C₁-C₂-alkyl)amino, and
the other of the radicals X and Y is
C₁-C₂-alkyl, C₁-C₂-haloalkyl, C₁-C₂-alkoxy, C₁-C₂-haloalkoxy or C₁-C₂-alkylthio, and
Z i s CH or N.

5. A process for the preparation of a compound of the formula (I) or of a salt thereof as claimed in one of claims 1 to 4, which comprises
a) reacting a compound of the formula (II) 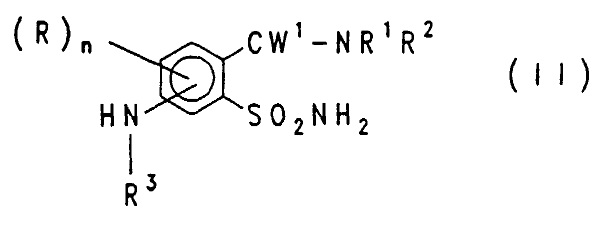 with a heterocyclic carbamate of the formula (III), 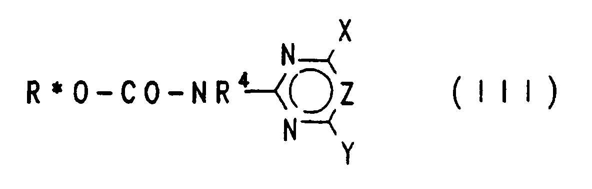 in which
R* is unsubstituted or substituted phenyl or C₁-C₄-alkyl, or
b) reacting a sulfonyl isocyanate of the formula (IV) 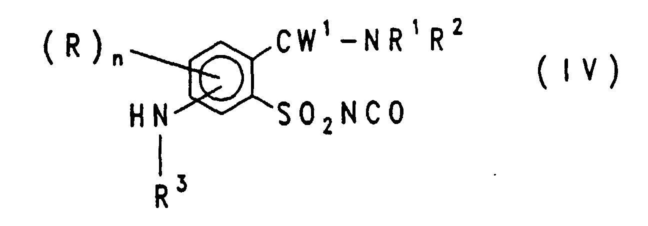 with a heterocyclic amine of the formula (V) 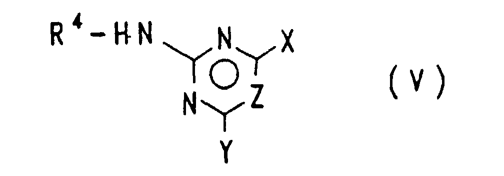 or
c) reacting a sulfonyl chloride of the formula (VI) 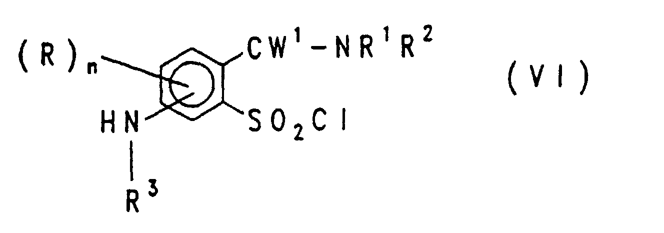 with a heterocyclic amine of the above formula (V) in the presence of a cyanate, or
d) reacting a sulfonamide of the above formula (II) with a (thio)isocyanate of the formula (VII) 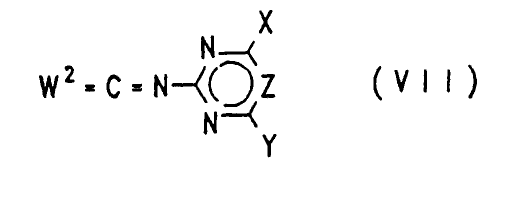 in the presence of a suitable base, such as, for example, potassium carbonate or triethylamine,
where, in the above formulae (II) to (VII), the radicals R, R¹, R², R³, R⁴, W¹, W², X, Y and Z and the index n are as defined in formula (I), and where compounds of the formula (I) in which W² is an oxygen atom are first obtained in variants a) - c).

6. A herbicidal or plant growth regulating composition, which comprises an active amount of at least one compound of the formula (I) or of one of its salts as claimed in one of claims 1 to 4 and formulation auxiliaries customary in plant protection.

7. A method of combating harmful plants or for regulating the growth of plants, which comprises applying an active amount of at least one compound of the formula (I) or of one of its salts as claimed in one of claims 1 to 4 to the harmful plants or to plants, plant seeds thereof or the surface on which the plants grow.

8. The use of a compound of the formula (I) or a salt thereof as claimed in one of claims 1 to 4 as a herbicide or plant growth regulator.

9. A compound of the formula (II), (IV) or (VI) as defined in claim 5.

10. A compound of the formula (VIII)* 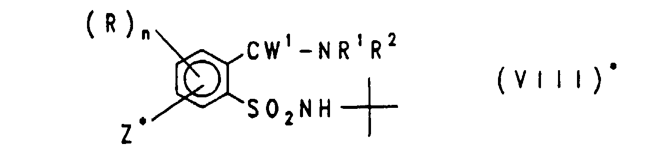
Z* = NHR³ (VIII)
Z* = NH₂ (IX)
Z* = NO₂ (X)
in which
Z* is NHR³, NH₂ or NO₂ and
R, n, R¹, R², R³ and W¹ are as defined in formula (I) as claimed in claim 1.

## Revendications

1. Composés de formule (I) et sels de ceux-ci : 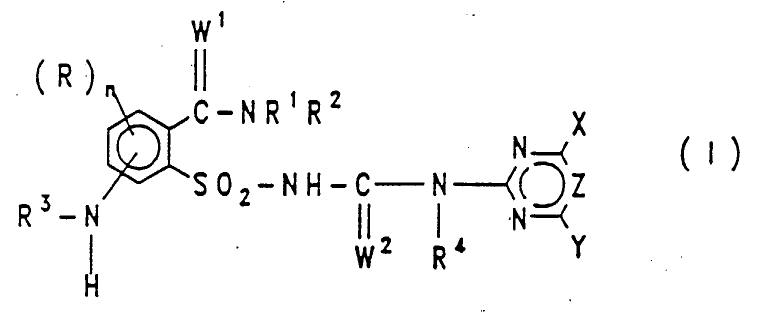 formule dans laquelle
W¹ représente un atome d'oxygène ou un atome de soufre,
W² représente un atome d'oxygène ou un atome de soufre,
n est 0, 1, 2 ou 3,
R représente un atome d'halogène, un groupe alkyle ou alkoxy, et est en effet indépendant des autres substituants R, lorsque n est supérieur à 1,
R¹ représente un atome d'hydrogène ou un groupe hydrocarboné ou hydrocarbonyloxy substitué ou non substitué,
R² représente un atome d'hydrogène ou un groupe hydrocarboné substitué ou non substitué,
ou le groupe
NR¹R² représente un noyau hétérocyclique avec 3 à 8 atomes de cycle, qui est non substitué ou substitué et contient l'atome d'azote du groupe NR¹R² comme atome d'hétérocycle et peut contenir d'autres atomes d'hétérocycle,
R³ représente un radical acyle,
R⁴ représente un atome d'hydrogène ou un groupe hydrocarboné aliphatique,
X, Y représentent indépendamment l'un de l'autre un atome d'halogène, un groupe alkyle en C₁-C₆, alkoxy en C₁-C₆, alkylthio en C₁-C₆, chacun des trois derniers radicaux cités étant non substitué ou substitué par un ou plusieurs résidus choisis parmi un atome d'halogène, un groupe alkoxy en C₁-C₄ et alkylthio en C₁-C₄, ou un groupe cycloalkyle en C₃-C₆, alcényle en C₂-C₆, alcynyle en C₂-C₆, alcényloxy en C₃-C₆ ou alcynyloxy en C₃-C₆ et
Z représente un groupe CH ou un atome d'azote.

2. Composés et sels de ceux-ci selon la revendication 1, caractérisés en ce que
R¹ représente un atome d'hydrogène ou un groupe alkyle, alcényle, alcynyle, alkoxy, alcénoxy, alcynoxy, cycloalkyle, cycloalcényle ou phényle, chacun des neuf derniers radicaux cités étant non substitué ou substitué et contenant en tout jusqu'à 24 atomes de carbone,
R² représente un atome d'hydrogène ou un groupe alkyle, alcényle, alcynyle, chacun des trois derniers radicaux cités étant non substitué ou substitué et contenant en tout jusqu'à 24 atomes de carbone,
ou le groupe
NR¹R² représente un noyau hétérocyclique non substitué ou substitué constitué de quatre jusqu'à huit atomes de cycle, le groupe contenant en tout jusqu'à 18 atomes de carbone,
R³ représente un groupe acyle ayant jusqu'à 24 atomes de carbone,
R⁴ représente un atome d'hydrogène ou un groupe alkyle, alcényle ou alcynyle, chacun des trois derniers radicaux cités contenant jusqu'à 12 atomes de carbone.

3. Composés et sels de ceux-ci selon la revendication 1, caractérisés en ce que
R¹ représente un atome d'hydrogène, un groupe alkyle en C₁-C₁₂, alcényle en C₂-C₁₂, alcynyle en C₂-C₁₂, alkoxy en C₁-C₁₂, alcénoxy en C₂-C₁₂, alcynoxy en C₂-C₁₂, cycloalkyle en C₃-C₈ ou cycloalcényle en C₅-C₈, chacun des huit derniers résidus cités étant non substitué ou substitué, par exemple par un ou plusieurs résidus choisis parmi un atome d'halogène, un groupe cyano, azido, CO-R⁵, OR⁶, NR⁷R⁸, SR⁹, SO-R¹⁰ et SO-R¹¹, ou phényle non substitué ou substitué,
R² représente un atome d'hydrogène, un groupe alkyle en C₁-C₁₂, alcényle en C₂-C₁₂ ou alcynyle en C₂-C₁₂, chacun des trois derniers résidus cités étant non substitué ou substitué par un ou plusieurs résidus choisis parmi un atome d'halogène, un groupe cyano, azido, COR¹², OR¹³, NR¹⁴R¹⁵, SR¹⁶, SO-R¹⁷ et SO-R¹⁸,
ou le groupe
NR¹R² représente un noyau hétérocyclique constitué de quatre jusqu'à huit atomes de cycle, qui peut contenir jusqu'à deux autres atomes d'hétérocycle choisis parmi N, O et S dans le noyau, et qui est non substitué ou substitué par un ou plusieurs résidus choisis parmi un atome d'halogène, un groupe alkyle en C₁-C₆, NO₂, N₃ et CN,
R³ représente CO-R¹⁹, CS-R²⁰, SO₂-R²¹, SO-R²¹ ou C (=NR²¹) -R¹⁹,
R⁴ représente un atome d'hydrogène, un groupe alkyle en C₁-C₃, alcényle en C₂-C₅ ou alcynyle en C₂-C₅
R⁵ représente un atome d'hydrogène, un groupe alkyle en C₁-C₅, alcényle en C₂-C₅, alcynyle en C₂-C₅, alkoxy en C₁-C₅, alcénoxy en C₂-C₅, alcynoxy en C₂-C₅, NR²²R²³ ou OH,
R⁶ représente un atome d'hydrogène, un groupe alkyle en C₁-C₅, halogénoalkyle en C₁-C₅, alcényle en C₂-C₅, halogénoalcényle en C₂-C₅, alcynyle en C₂-C₅, halogénoalcynyle en C₂-C₅,
R⁷ représente un atome d'hydrogène, un groupe alkyle en C₁-C₅, alcényle en C₂-C₅, alcynyle en C₂-C₅, alkoxy en C₁-C₅, CO-CH₃, CO-H, COOCH₃,
R⁸ représente un atome d'hydrogène, un groupe alkyle en C₁-C₅, alcényle en C₂-C₅, alcynyle en C₂-C₅,
ou le groupe
NR⁷R⁸ représente un noyau hétérocyclique constitué de quatre jusqu'à huit atomes de cycle, qui peut contenir jusqu'à deux autres atomes d'hétérocycle, choisis parmi N, O et S dans le noyau, et qui est non substitué ou substitué par un ou plusieurs résidus choisis parmi un atome d'halogène, un groupe alkyle en C₁-C₆, NO₂, N₃ et CN,
R⁹ représente un atome d'hydrogène, un groupe alkyle en C₁-C₅, alcényle en C₂-C₅, alcynyle en C₂-C₅, halogénoalkyle en C₁-C₅, halogénoalcényle en C₂-C₅, halogénoalcynyle en C₂-C₅,
R¹⁰ représente un groupe alkyle en C₁-C₅, alcényle en C₂-C₅, alcynyle en C₂-C₅, halogénoalkyle en C₁-C₅, halogénoalcényle en C₂-C₅, halogénoalcynyle en C₂-C₅,
R¹¹ représente un groupe alkyle en C₁-C₅, alcényle en C₂-C₅, alcynyle en C₂-C₅, halogénoalkyle en C₁-C₅, halogénoalcényle en C2-C5, halogénoalcynyle en C₂-C₅, alkoxyalkyle en C₁-C₅, cycloalkyle en C₃-C₈ ou cycloalcényle en C₅-C₈,
R¹² représente un groupe analogue à R⁵,
R¹³ un groupe analogue à R⁶,
R¹⁴ un groupe analogue à R⁷,
R¹⁵ un groupe analogue à R⁸,
ou le groupe
NR¹⁴R¹⁵ représente un groupe analogue à NR⁷R⁸,
R¹⁶ un groupe analogue à R⁹,
R¹⁷ un groupe analogue à R¹⁰,
R¹⁸ un groupe analogue à R¹¹,
R¹⁹ représente un atome d'hydrogène, un groupe alkyle en C₁-C₁₂, alcényle en C₂-C₁₂, alcynyle en C₂-C₁₂, alkoxy en C₁-C₁₂, alkylthio en C₁-C₁₂, alcénoxy en C₂-C₁₂, alcynoxy en C₂-C₁₂, cycloalkyle en C₃-C₈, cycloalcényle en C₅-C₈, alkylamino en C₁-C₁₂, di-(alkyle en C₁-C₆)-amino, N-alkoxy en C₁-C₄-N-alkyle en C₁-C₄-amino, chacun des 13 derniers résidus cités étant non substitué ou substitué par un ou plusieurs résidus choisis parmi un atome d'halogène, un groupe cyano, azido, CO-R²⁴, OR²⁵, NR²⁶R²⁷, SR²⁸, SOR²⁹ et SO₂R³⁰, ou un groupe phényle, phénoxy ou phénylamino non substitué ou substitué,
R²⁰ un groupe analogue à R¹⁹,
R²¹ un groupe analogue à R¹¹,
R²² représente un atome d'hydrogène, un groupe alkyle en C₁-C₅, alcényle en C₂-C₅, alcynyle en C₂-C₅, alkoxy en C₁-C₅, alcénoxy en C₂-C₅, alcynoxy en C₂-C₅, chacun des derniers résidus cités étant non substitué ou substitué par un ou plusieurs résidus choisis parmi un atome d'halogène, un groupe cyano, azido, amino, amino mono et disubstitué, alkyle en C₁-C₃, alkoxy en C₁-C₃, alkylsulfonyle en C₁-C₃, alkylsulfinyle en C₁-C₃ et alkylthio en C₁-C₃,
R²³ représente un atome d'hydrogène, un groupe alkyle en C₁-C₅, alcényle en C₂-C₅, alcynyle en C₂-C₅, alkoxy en C₁-C₅, alcénoxy en C₂-C₅, chacun des 5 derniers résidus cités étant non substitué ou substitué par un ou plusieurs résidus choisis parmi un atome d'halogène, un groupe cyano, azido, amino, amino mono et disubstitué, alkoxy en C₁-C₃, alkylthio en C₁-C₃, alkylsulfonyle en C₁-C₃ et alkylsulfinyle en C₁-C₃,
ou le groupe
NR²²R²³ représente un noyau hétérocyclique analogue à NR⁷R⁸,
R²⁴ représente un atome d'hydrogène, un groupe alkyle en C₁-C₃, alkoxy en C₁-C₃, NH₂, amino mono et disubstitué,
R²⁵ représente un atome d'hydrogène, un groupe alkyle en C₁-C₅, alcényle en C₂-C₅, alcynyle en C₂-C₅, halogénoalkyle en C₁-C₅, halogénoalcényle en C₂-C₅, halogénoalcynyle en C₂-C₅,
R²⁶ représente un atome d'hydrogène, un groupe alkyle en C₁-C₃, alkoxy en C₁-C₃, CO-CH₃, CO-H,
R²⁷ représente un atome d'hydrogène, un groupe alkyle en C₁-C₃,
ou le groupe
NR²⁶R²⁷ représente un groupe analogue à NR²²R²³,
R²⁸ représente un atome d'hydrogène, un groupe alkyle en C₁-C₃, halogénoalkyle en C₁-C₃, alcényle en C₂-C₅, halogénoalcényle en C₂-C₅, alcynyle en C₂-C₅, halogénoalcynyle en C₂-C₅,
R²⁹ représente un groupe alkyle en C₁-C₅, halogénoalkyle en C₁-C₅, alcényle en C₂-C₅, halogénoalcényle en C₂-C₅, alcynyle en C₂-C₅, halogénoalcynyle en C₂-C₅,
R³⁰ un groupe analogue à R²⁹,
X, Y représentent indépendamment l'un de l'autre un atome d'halogène, un groupe alkyle en C₁-C₄, alkoxy en C₁-C₄, alkylthio en C₁-C₄, chacun des trois derniers radicaux cités étant non substitué ou substitué par un ou plusieurs résidus choisis parmi un atome d'halogène, un groupe alkoxy en C₁-C₄ et alkylthio en C₁-C₄,
un groupe mono- ou di-(alkyle en C₁-C₄)amino, cycloalkyle en C₃-C₆, alcényle en C₃-C₅ ou alcynyloxy en C₃-C₅.

4. Composés et sels de ceux-ci selon l'une des revendications 1 à 3, caractérisés en ce que le groupe NHR³ se trouve en position méta sur le noyau phényle par rapport au groupe SO₂ et en position para par rapport au groupe CW¹-NR¹R² et en ce que
W¹ représente un atome d'oxygène,
W² représente un atome d'oxygène,
n est 0,
R¹ représente un atome d'hydrogène, un groupe alkyle en C₁-C₂, alkoxy en C₁-C₂, phényle, qui est non substitué ou substitué par un ou plusieurs résidus choisis parmi un atome d'halogène, un groupe alkyle en C₁-C₂, alkoxy en C₁-C₂, halogénoalkyle en C₁-C₂ et halogénoalkoxy en C₁-C₂,
R² représente un atome d'hydrogène, un groupe alkyle en C₁-C₂ ou alkoxy en C₁-C₂
ou le groupe
NR¹R² représente un noyau hétérocyclique constitué de 5 ou 6 atomes de cycle, qui peut contenir jusqu'à un autre atome d'hétérocycle choisi entre N et O dans le noyau, et qui est non substitué ou substitué par un ou plusieurs résidus appartenant au groupe alkyle en C₁-C₂,
R³ représente CO-R¹⁹, CS-R²⁰ ou SO₂-R²¹,
R⁴ représente un atome d'hydrogène ou un groupe CH₃,
R¹⁹ représente un atome d'hydrogène, un groupe alkyle en C₁-C₄, alcényle en C₂-C₄, alcynyle en C₂-C₄, alkoxy en C₁-C₄, alkylthio en C₁-C₄, alcénoxy en C₂-C₄, alcynoxy en C₂-C₄, cycloalkyle en C₃-C₆, alkylamino en C₁-C₄, di-(alkyle en C₁-C₄)-amino, N-alkoxy en C₁-C₂-N-alkyle en C₁-C₂-amino, chacun des 10 derniers résidus cités étant non substitué ou substitué par un ou plusieurs résidus choisis entre un atome d'halogène et un groupe alkoxy en C₁-C₄, ou un groupe phényle ou phénoxy, qui est non substitué ou substitué par des résidus choisis parmi un atome d'halogène, un groupe alkyle en C₁-C₂, alkoxy en C₁-C₂, halogénoalkyle en C₁-C₂ et halogénoalkoxy en C₁-C₂,
R²⁰ un groupe analogue à R¹⁹ et
R²¹ un groupe alkyle en C₁-C₃, halogénoalkyle en C₁-C₃, alkoxy en C₁-C₃-alkyle en C₁-C₂.
un des résidus X et Y
représente un atome d'halogène, un groupe alkyle en C₁-C₂, alkoxy en C₁-C₂, alkylthio en C₁-C₂, chacun des trois derniers radicaux cités étant non substitué ou substitué par un ou plusieurs résidus choisis parmi un atome d'halogène, un groupe alkoxy en C₁-C₂ et alkylthio en C₁-C₂, ou un groupe mono- ou di-(alkyle en C₁-C₂)amino,
l'autre des résidus X et Y
représente un groupe alkyle en C₁-C₂, halogénoalkyle en C₁-C₂, alkoxy en C₁-C₂, halogénoalkoxy en C₁-C₂ ou alkylthio en C₁-C₂ et
Z représente un groupe CH ou un atome d'azote.

5. Procédé de préparation des composés de formule (I) ou des sels de ceux-ci selon l'une des revendications 1 à 4, caractérisé en ce que
a) on met à réagir un composé de formule (Il) 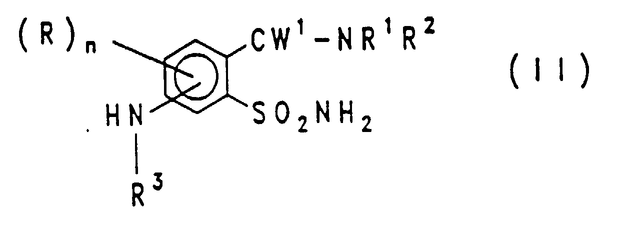 avec un carbamate hétérocyclique de formule (III) 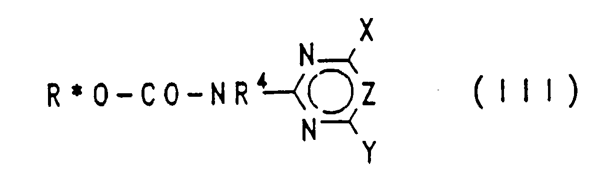 dans lequel R* représente un groupe phényle non substitué ou substitué, ou alkyle en C₁-C₄, ou
b) on met à réagir un isocyanate de sulfonyle de formule (IV) 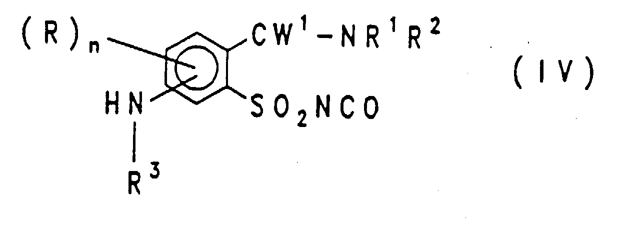 avec une amine hétérocyclique de formule (V) 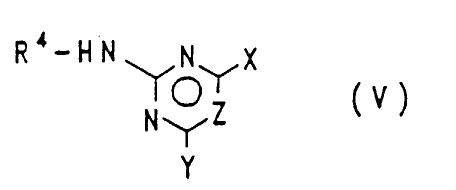 ou
c) on met à réagir un sulfochlorure de formule (VI) 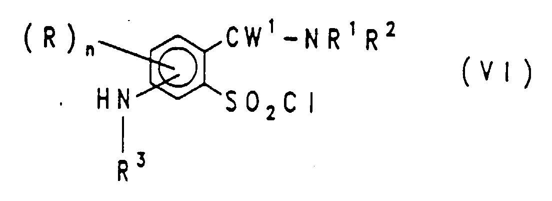 avec une amine hétérocyclique de formule (V) citée en présence d'un cyanate ou
d) on met à réagir un sulfonamide de formule (Il) citée avec un (thio)isocyanate de formule (VII) 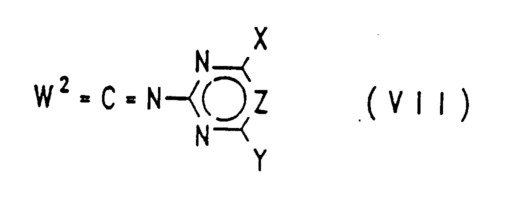 en présence d'une base appropriée, comme par exemple le carbonate de calcium ou la triéthylamine,
formules (Il) à (VII) ci-dessus dans lesquelles les groupes R, R¹, R², R³, R⁴, W¹, W², X, Y et Z ainsi que l'indice n sont définis comme dans la formule (I) et variantes de procédé a) - c) dans lesquelles on obtient d'abord des composés de formule (I), dans lesquels W² représente un atome d'oxygène.

6. Agent herbicide ou régulateur de croissance des végétaux, caractérisé en ce qu'il contient au moins une quantité efficace d'un composé de formule (I) ou de l'un de ses sels selon l'une des revendications 1 à 4 et des additifs de formulation usuels dans la protection des plantes.

7. Procédé pour combattre les plantes nuisibles ou pour réguler la croissance de végétaux, caractérisé en ce que, l'on applique une quantité efficace d'au moins un composé de formule (I) ou d'un de ses sels selon l'une des revendications 1 à 4 sur les plantes nuisibles et les végétaux, leurs semences ou la surface sur laquelle poussent les végétaux.

8. Utilisation des composés de formule (I) ou de leurs sels selon l'une des revendications 1 à 4 comme herbicides ou régulateurs de croissance de végétaux.

9. Composés de formules (Il), (IV), (VI), tels qu'ils sont définis dans la revendication 5.

10. Composés de formule (VIII)* 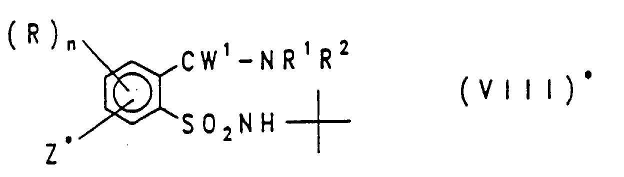
Z* = NHR³ (VIII)
Z* = NH₂ (IX)
Z* = NO₂ (X)
dans laquelle
Z* représente un groupe NHR³, NH₂ ou NO₂ et
R, n, R¹, R², R³ et W¹ sont définis comme dans la formule (I) selon la revendication 1.
